# EUROPEAN PATENT APPLICATION

(11) **EP 4 645 322 A1**
(43) Date of publication of application: **05.11.2025**
(21) Application number: 23912256.7
(22) Date of filing: 27.12.2023
(51) Int. Cl.: G16C 20/30, G06N 20/00, G06Q 50/10

(54) **INFORMATION PROCESSING SYSTEM AND PROGRAM**

(30) Priority: 28.12.2022 JP 2022212206
(71) Applicant: Crowdchem Co., Ltd., Tokyo 143-0023 (JP)
(72) Inventor: IKEBATA Hisaki, Tokyo 143-0023 (JP)
(74) Representative: EIP
(86) International application number: PCT/JP2023/046964
(87) International publication number: WO 2024/143478

(57) **Abstract**

Comprising at least one processor obtaining a combination of information identifying each of the raw materials received from the user and the amount of each of the raw materials, and obtaining a predicted value of a physical property of the property name to be predicted for a composition comprising each of the raw materials by inputting into a first machine learning model at least one of the chemical fingerprints, SMILES strings or chemical graph structure data or product name or substance name corresponding to each of the raw materials and the amount of each of the raw materials, or by inputting into a second machine learning model a set of values based on at least one of the chemical fingerprints, SMILES strings or chemical graph structure data or product name or substance name corresponding to each of the raw materials and the amount of each of the raw materials,
wherein the first machine learning model is a model in which parameters are adjusted so that it can predict outputs from inputs by means of a learning data set that takes as inputs at least one of the chemical fingerprints, SMILES strings or chemical graph structure data or product names or substance names corresponding to each of the raw materials and the amount of each of the said raw materials, and as takes as outputs physical property values of the target property names to be predicted, and the second machine learning model is a model in which parameters are adjusted so that it can predict outputs from inputs by means of a learning data set that takes as inputs a set of values based on at least one of the chemical fingerprints, SMILES strings or chemical graph structure data or product names or substance names corresponding to each of the raw materials and the amount of each of the raw materials, and takes as outputs physical property values of the target property names to be predicted.

## Description

### Technical Field

The present invention relates to an information processing system and a program.

### Background Art

Methods for predicting physical property data of compositions have been developed. For example, in Patent Document 1, it is described that the physical property data is machine-learned by a prediction module of a computer using the physical property data on multiple vulcanized rubber compositions, the identifying names of raw materials in the vulcanized rubber compositions, the blending ratio of the raw materials, and the information on the processing conditions. In addition, Patent Document 1 describes having a machine-learned prediction module predict the physical property data of a vulcanized rubber composition to be predicted, using the names of the constituent raw materials constituting the unvulcanized rubber composition before vulcanization of the vulcanized rubber composition to be predicted, the blending ratio of the constituent raw materials, and the processing conditions in the processing to prepare the vulcanized rubber composition to be predicted.

### Citation List

### Patent Literature

Patent Literature 1: JP2020-038495A1

### Non-Patent Literature

Non-Patent Literature 1:
   https://arxiv.org/abs/1712.02034
Non-Patent Literature 2: https://docs.dgl.ai/en/0.8.x/guide/training-graph.html

### Summary of Invention

### Means for solving the Problem

An information processing system according to a 1st aspect of the present invention, comprises
at least one processor obtaining a combination of information identifying each of the raw materials received from the user and the amount of each of the raw materials, and obtaining a predicted value of a physical property of the property name to be predicted for a composition comprising each of the raw materials by inputting into a first machine learning model at least one of the chemical fingerprints, SMILES strings or chemical graph structure data or product name or substance name corresponding to each of the raw materials and the amount of each of the raw materials, or by inputting into a second machine learning model a set of values based on at least one of the chemical fingerprints, SMILES strings or chemical graph structure data or product name or substance name corresponding to each of the raw materials and the amount of each of the raw materials,
wherein the first machine learning model is a model in which parameters are adjusted so that it can predict outputs from inputs by means of a learning data set in which at least one of the chemical fingerprints, SMILES strings or chemical graph structure data or product names or substance names corresponding to each of the raw materials and the amount of each of the said raw materials are input, and physical property value of the target property name to be predicted is output, and the second machine learning model is a model in which parameters are adjusted so that it can predict outputs from inputs by means of a learning data set in which a group of numerical values based on at least one of the chemical fingerprints, SMILES strings or chemical graph structure data or product names or substance names corresponding to each of the raw materials and the amount of each of the raw materials is input, and physical property value of the target property name to be predicted is output.

An information processing system according to a 2nd aspect of the present invention, in the 1st aspect of the present invention, wherein the first machine learning model or the second machine learning model is constructed by collectively learning a plurality of physical properties of raw materials used in multiple fields.

An information processing according to a 3rd aspect of the present invention, in the 1st or the 2nd aspect of the present invention, wherein the raw materials include additives, and the input of the first machine learning model or the second machine learning model includes the type of raw material or a group of numerical values based on the type, and the processor further inputs the type of raw material or the group of numerical values based on the type acquired from the user into the first machine learning model or the second machine learning model, thereby obtaining a predicted value of the physical property value of the prediction target physical property name for a composition composed of each of the raw materials.

An information processing according to a 4th aspect of the present invention, in the 1st or 2nd aspect of the present invention, wherein the input of the first machine learning model or the second machine learning model further includes the physical properties of the raw materials or a group of numerical values based on the physical properties, and the processor further inputs the physical properties of the raw materials or a group of numerical values based on the physical properties obtained from the user into the first machine learning model or the second machine learning model, thereby obtaining a predicted value of the characteristic value of the prediction target physical property name for a composition composed of each of the raw materials.

An information processing according to a 5th aspect of the present invention, in any one of the 1st to the 4th aspect of the present invention, wherein the input of the first machine learning model or the second machine learning model includes the characteristics of raw materials or a group of numerical values based on the characteristics, and the processor further inputs the characteristics of the raw materials or the group of numerical values based on the characteristics obtained from the user into the first machine learning model or the second machine learning model, thereby obtaining a predicted value of the physical property value of the prediction target physical property name for a composition composed of each of the raw materials.

An information processing system according to a 6th aspect of the present invention, in any one of the 1st to 5th aspect of the present invention, wherein the input of the first machine learning model or the second machine learning model includes information about process conditions and/or equipment used in the process or a group of numerical values based on the information, and the processor further inputs the information about the process conditions and/or equipment used in the process acquired from the user or a group of numerical values based on the information into the first machine learning model or the second machine learning model, thereby obtaining a predicted value of the physical property value of the prediction target physical property name for a composition composed of each of the raw materials.

An information processing system according to a 7th aspect of the present invention, in any one of the 1st to 6th aspect of the present invention, wherein the processor extracts at least one raw material candidate based on at least one of the raw material product name, raw material manufacturer name, raw material category, and raw material uses received from the user, and outputs information for displaying the extracted raw material candidate in a selectable manner.

An information processing system according to the 8th aspect of the present invention, comprises:
at least one processor,
wherein the processor creates a child population of the next generation according to a genetic algorithm by regarding a combination of raw materials and the amounts of each of the raw materials as a parent population,
calculates a fitness function value using the combination of raw materials and the amounts of each of the raw materials indicated by the created child population of the next generation,
repeates the process of creating a child population of the next generation until a termination condition based on a physical property value range specified by the user is satisfied, and when the termination condition is satisfied, outputs information including the combination of raw materials indicated by each of the child populations of the next generation created last and the amounts of each of the raw materials.

An information processing system according to a 9th aspect of the present invention, in the 8th aspect of the present invention, wherein the processor acquires at least one set of desired property value ranges input by a user for a desired property name, and inputs at least one of a chemical fingerprint, a SMILES character string, or chemical graph structure data, or a product name or a substance name corresponding to each of the raw materials included in the initial combination of raw materials and an amount of each of the raw materials into a first machine learning model, or inputs a group of numerical values based on at least one of a chemical fingerprint, a SMILES character string, or chemical graph structure data, or a product name or a substance name corresponding to each of the raw materials and an amount of each of the raw materials into a second machine learning model, thereby acquiring a predicted value of a property value of the physical property name,
narrows down the obtained predicted values of the physical properties to combinations of raw materials and the amounts of each of the raw materials that satisfy the desired range of the physical properties specified by the user,
considers the narrowed down combinations of raw materials and the amounts of each of the raw materials as a parent population, and creates a child population of the next generation according to a genetic algorithm,
the first machine learning model is a model in which parameters are adjusted so that the output can be predicted from the input using a learning data set in which at least one of the chemical fingerprints, SMILES character strings, chemical graph structure data, product names, or substance names corresponding to each of the raw materials and the amounts of each of the raw materials are input, and the physical property value of the prediction target physical property name is output,
the second machine learning model is a model in which parameters are adjusted so that an output can be predicted from an input using a learning data set in which a group of numerical values obtained by converting at least one of a chemical fingerprint, a SMILES character string, a chemical graph structure data, a product name, or a substance name corresponding to each raw material and the amount of each raw material is input, and a physical property value of a physical property name is output.

An information processing system according to a 10th aspect of the present invention, in the 9th aspect of the present invention, wherein the first machine learning model or the second machine learning model is constructed for each physical property name, and the processor obtains a predicted value of the physical property value of the prediction target physical property name by using the first machine learning model or the second machine learning model corresponding to the physical property name received from the user.

An information processing system according to a 11th aspect of the present invention, in the 9th or 10th aspect of the present invention, wherein the processor further outputs a predicted value of the property value of the desired property name exhibited by each of the offspring population of the next generation finally produced.

An information processing system according to a 12th aspect of the present invention, in the 8th or 11th aspect of the present invention, wherein when one of the outputted raw materials is selected by the user, the processor outputs contact information of a sales company that sells the raw material and/or detailed information of the raw material.

An information processing system according to a 13th aspect of the present invention, comprises:
at least one storage device in which data relating to working examples of patent documents are stored; and
at least one processor,
wherein the processor extracts, from among the working examples stored in the storage device, a working example that use raw materials designated by a user and/or a working example that do not use the input raw materials among raw materials with similar physical property values realized by the combination of the raw materials, and outputs information for displaying a list of applicants or patent holders of patent documents in which the extracted working examples are described.

An information processing system according to a 14th aspect of the present invention, in the 13th aspect of the present invention, wherein after extracting the working examples, the processor narrows down the working examples to raw materials that have similar physical property values when the target raw material designated by the user is replaced with other raw material.

An information processing system according to a 15th aspect of the present invention, in the 13th aspect of the present invention, wherein when narrowing down raw materials whose physical property values are similar when a target raw material designated by a user is replaced with other raw material, the processor inputs a pair of the other raw material and the amount of the other raw material into a trained machine learning model of a first embodiment to determine predicted values of the physical property values of the predicted target physical property name, and compares each of the predicted values of the physical property values with the physical property values of the target raw material to extract raw materials whose predicted physical property values are equal to or greater than or similar to the physical property values of the raw material designated by the user.

An information processing system according to a 16th aspect of the present invention, in the 13th to 15th aspect of the present invention, wherein the list includes similar ingredients described in the extracted examples as ingredients to be substituted.

An information processing system according to a 17th aspect of the present invention, comprises:
at least one processor,
wherein the at least one processor acquires target physical property values specified by a user,
acquires a range of blend amounts specified by the user,
determines multiple candidate blend amounts within the range of blend amounts specified by the user for each of the candidate raw materials, predicts physical property values for all combinations of a set of candidate raw materials and each of the candidate blend amounts of the candidate raw materials, and
outputs the set of candidate raw materials and each of the candidate raw materials based on a comparison between the target physical property values and each of the predicted physical property values.

An information processing system according to a 18th aspect of the present invention, in the 17th aspect of the present invention, wherein the at least one processor obtains a substance category designated by a user, and extracts each of the raw materials belonging to the substance category designated by the user as the candidate raw materials.

An information processing system according to a 19th aspect of the present invention, comprises:
at least one processor,
wherein the at least one processor executes the steps of:
   determining a plurality of candidate values for the amount of other raw material in a range specified by a user when a portion of the raw materials constituting a target composition is replaced with other raw material specified by a user;
   determining predicted values of physical properties for all of the determined plurality of candidate values for the amount; and
   outputting information for presenting predicted values of physical properties in correspondence with each of the plurality of candidate values for the amount,
   in the step of determining the predicted value of the physical property, the at least one processor inputs at least one of a SMILES character string, a chemical graph structure data, a product name, or a substance name corresponding to each of the raw materials and an amount of each of the raw materials to a first machine learning model, or inputs a group of numerical values based on at least one of a chemical fingerprint, a SMILES character string, a chemical graph structure data, a product name, or a substance name corresponding to each of the raw materials and an amount of each of the raw materials to a second machine learning model, thereby obtaining a predicted value of the physical property,
   the first machine learning model is a model in which parameters are adjusted so that the output can be predicted from the input using a learning data set in which at least one of a SMILES character string, chemical graph structure data, product name, or substance name corresponding to each raw material and the amount of each raw material are input, and the physical property value of the prediction target physical property name is output,
   the second machine learning model is a model in which parameters are adjusted so that an output can be predicted from an input using a learning data set in which a group of numerical values obtained by converting at least one of a chemical fingerprint, a SMILES character string, or chemical graph structure data, or a product name or a substance name corresponding to each raw material and the amount of each raw material is input, and a physical property value of a prediction target physical property name is output.

A program according to a 20th aspect of the present invention, is a program for causing a computer capable of referring to at least one storage device that stores
a first machine learning model is a model in which parameters are adjusted so that it can predict outputs from inputs by means of a learning data set in which at least one of the chemical fingerprints, SMILES strings or chemical graph structure data or product names or substance names corresponding to each of the raw materials and the amount of each of the said raw materials are input, and physical property value of the target property name to be predicted is output, or
a second machine learning model is a model in which parameters are adjusted so that it can predict outputs from inputs by means of a learning data set in which a group of numerical values based on at least one of the chemical fingerprints, SMILES strings or chemical graph structure data or product names or substance names corresponding to each of the raw materials and the amount of each of the raw materials is input, and physical property value of the target property name to be predicted is output,
to execute obtaining a combination of information identifying each of the raw materials received from the user and the amount of each of the raw materials, and obtaining a predicted value of a physical property of the property name to be predicted for a composition comprising each of the raw materials by inputting into a first machine learning model at least one of the chemical fingerprints, SMILES strings or chemical graph structure data or product name or substance name corresponding to each of the raw materials and the amount of each of the raw materials, or by inputting into a second machine learning model a set of values based on at least one of the chemical fingerprints, SMILES strings or chemical graph structure data or product name or substance name corresponding to each of the raw materials and the amount of each of the raw materials.

A program according to a 21st aspect of the present invention, is a program for causing a computer to execute
creating a child population of the next generation according to a genetic algorithm by regarding a combination of raw materials and the amounts of each of the raw materials as a parent population
calculating a fitness function value using the combination of raw materials and the amounts of each of the raw materials indicated by the created child population of the next generation,
repeating the process of creating a child population of the next generation until a termination condition based on a physical property value range specified by the user is satisfied, and when the termination condition is satisfied, outputting information including the combination of raw materials indicated by each of the child populations of the next generation created last and the amounts of each of the raw materials.

A program according to a 22nd aspect of the present invention, is a program for causing a computer capable of referring to at least one storage device in which data related to a working example of the patent document is stored, to execute
extracting, from among the working examples stored in the storage device, a working example that use raw materials designated by a user and/or a working example that do not use the input raw materials among raw materials with similar physical property values realized by the combination of the raw materials, and
outputting information for displaying a list of applicants or patent holders of patent documents in which the extracted working examples are described.

A program according to a 23rd aspect of the present invention, is a program for causing a computer to execute
acquiring target physical property values specified by a user,
acquiring a range of blend amounts specified by the user,
determining multiple candidate blend amounts within the range of blend amounts specified by the user for each of the candidate raw materials, predicts physical property values for all combinations of a set of candidate raw materials and each of the candidate blend amounts of the candidate raw materials, and
outputting the set of candidate raw materials and each of the candidate raw materials based on a comparison between the target physical property values and each of the predicted physical property values.

A program according to a 24th aspect of the present invention, is a program for causing a computer to execute
determining a plurality of candidate values for the amount of other raw material in a range specified by a user when a portion of the raw materials constituting a target composition is replaced with other raw material specified by a user;
determining predicted values of physical properties for all of the determined plurality of candidate values for the amount; and
outputting information for presenting predicted values of physical properties in correspondence with each of the plurality of candidate values for the amount,
in the step of determining the predicted value of the physical property, the at least one processor inputs at least one of a SMILES character string, a chemical graph structure data, a product name, or a substance name corresponding to each of the raw materials and an amount of each of the raw materials to a first machine learning model, or inputs a group of numerical values based on at least one of a chemical fingerprint, a SMILES character string, a chemical graph structure data, a product name, or a substance name corresponding to each of the raw materials and an amount of each of the raw materials to a second machine learning model, thereby obtaining a predicted value of the physical property,
the first machine learning model is a model in which parameters are adjusted so that the output can be predicted from the input using a learning data set in which at least one of a SMILES character string, chemical graph structure data, product name, or substance name corresponding to each raw material and the amount of each raw material are input, and the physical property value of the prediction target physical property name is output,
the second machine learning model is a model in which parameters are adjusted so that an output can be predicted from an input using a learning data set in which a group of numerical values obtained by converting at least one of a chemical fingerprint, a SMILES character string, or chemical graph structure data, or a product name or a substance name corresponding to each raw material and the amount of each raw material is input, and a physical property value of a prediction target physical property name is output.

### Brief Description of Drawings

Figure 1 is a schematic configuration diagram of an information processing system common to each embodiment.
Figure 2 is a schematic configuration diagram of a terminal common to each embodiment.
Figure 3 is a schematic configuration diagram of a computer system common to each embodiment.
Figure 4 is an example of a table stored in a storage device of a computer system.
Figure 5 is a diagram showing an example of a screen for selecting raw materials displayed on the terminal of the first embodiment.
Figure 6 is an example of a screen subsequent to that shown in FIG. 5.
Figure 7 is a diagram showing an example of a screen for selecting raw materials displayed on a terminal according to the second embodiment.
Figure 8 is an example of a screen following that of FIG. 7.
Figure 9 is a flowchart showing an example of the flow of a search process according to the second embodiment.
Figure 10 is a flowchart showing an example of the flow of a search process using a genetic algorithm in step S250 of FIG. 9.
Figure 11 is a diagram illustrating an example of a screen transition on a terminal according to the third embodiment.
Figure 12 is an example of a screen subsequent to that shown in FIG. 11.
Figure 13 is a flowchart showing an example of a processing flow when a sales destination candidate button according to the third embodiment is pressed.
Figure 14 is a diagram for explaining a grid search of raw material types and blend amounts.
Figure 15A is a diagram illustrating an example of a screen of a terminal according to the fourth embodiment.
Figure 15B is a diagram showing an example of a patent document search screen in the fourth embodiment.
Figure 16 is an example of an embodiment selection screen in the fourth embodiment.
Figure 17 is an example of a screen that appears when the "AI product search" button B121 associated with embodiment 1 of the selected patent document is pressed on the screen G12 of FIG. 16.
Figure 18 is an example of a screen that appears when, for example, the "Change" button B131 is pressed on the screen G13 of FIG. 17.
Figure 19 is an example of a screen showing a prediction result of a physical property value according to the fourth embodiment.

### Description of Embodiments

Each embodiment will be described below with reference to the drawings. However, more detailed explanations than necessary may be omitted. For example, detailed explanations of matters that are already well known or duplicate explanations of substantially identical configurations may be omitted. This is to avoid unnecessarily lengthy explanations and to make the following explanation easier to understand for those skilled in the art.

### <First problem>

The problem is that it is difficult to obtain the physical properties of the composition obtained by combining raw materials.

One embodiment of the present invention has been made in consideration of the above problems, and its first issue is to make it easier to obtain the physical property values of a composition obtained by combining raw materials.

### <Second problem>

There is also the problem that it is difficult for users to obtain a combination of raw materials that satisfies the material properties they desire. One embodiment of the present invention has been made in consideration of the above problem, and its second issue is to make it easier to obtain a combination of raw materials that satisfies the material properties they desire.

### <Third problem>

There is also the problem that it is difficult for raw material manufacturers and the like to search for companies that may be willing to use the target raw materials. One embodiment of the present invention has been made in consideration of the above problem, and its third problem is to make it easier to search for companies that may be willing to use the target raw materials.

Each embodiment has been made in consideration of the above problems, and aims to provide an information processing system and a program that can improve at least one of these problems.

### <First embodiment>

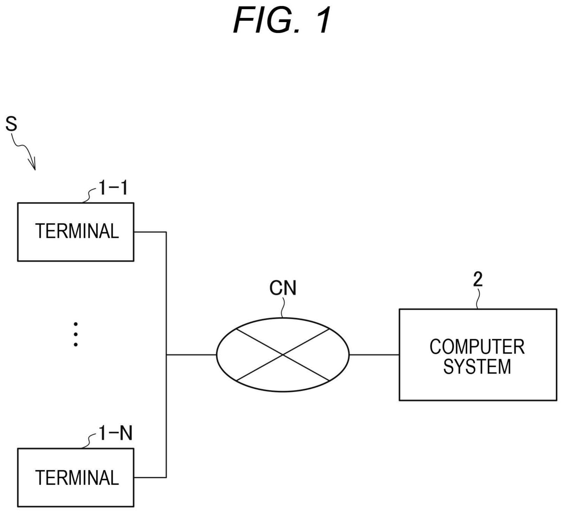

In the first embodiment, an information processing system that solves the first problem will be described. FIG. 1 is a schematic diagram of an information processing system common to each embodiment. As shown in FIG. 1, the information processing system S includes terminals 1-1, ..., 1-N (N is a natural number) used by users and a computer system 2. Each of the terminals 1-1, ..., 1-N is communicatively connected to the computer system 2 via a communication circuit network CN. The terminals are, for example, computers such as smartphones, tablet terminals, notebook computers, or personal computers. Hereinafter, the terminals 1-1, ..., 1-N will be collectively referred to as terminals 1.

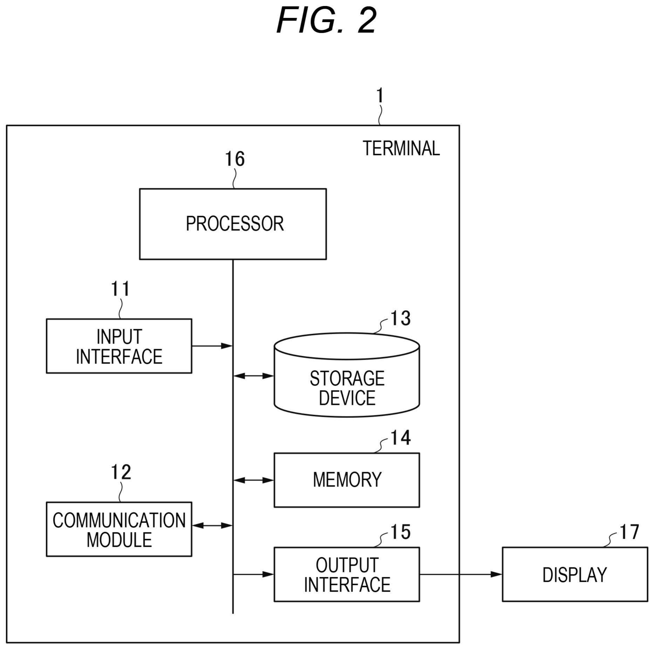

FIG. 2 is a schematic diagram of a terminal common to all embodiments. As shown in FIG. 2, terminal 1, as an example, includes an input interface 11, a communication module 12, a storage device 13, a memory 14, an output interface 15, and a processor 16. Note that, as one embodiment, terminal 1 is described as including one processor 16, but there may be multiple processors, i.e., one or more processors. Also, as one embodiment, terminal 1 is described as including one storage device 13, but there may be multiple processors, i.e., one or more storage devices.

The input interface 11 accepts input from the user of the terminal 1 and outputs an input signal corresponding to the accepted input to the processor 16. The communication module 12 is connected to the communication network CN and communicates with the computer system 2. This communication may be wired or wireless.

The storage device 13 is, for example, a storage, and stores programs and various data to be read and executed by the processor 16. The memory 14 temporarily holds the data and programs. The memory 14 is a volatile memory, for example, a RAM (Random Access Memory).

The output interface 15 can be connected to an external display 17 and can output signals to the display 17. The processor 16 loads a program from the storage device 13 into the memory 14 and performs various processes by executing a series of instructions contained in the program.

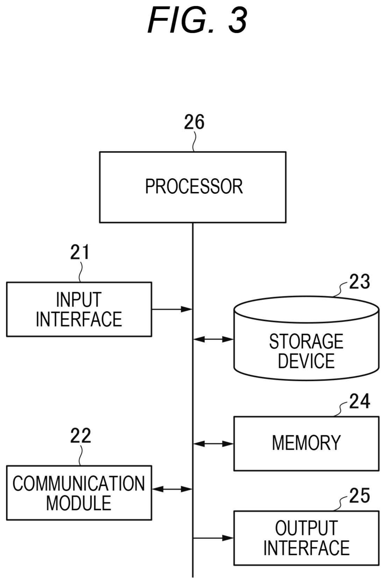

FIG. 3 is a schematic diagram of a computer system common to all embodiments.
As shown in FIG. 3, computer system 2, as an example, includes an input interface 21, a communications module 12, a storage device 23, a memory 24, an output interface 25, and a processor 26. Note that, as one embodiment, computer system 2 is described as including one processor 26, but there may be multiple processors, i.e., one or more processors. Also, as one embodiment, computer system 2 is described as including one storage device 13, but there may be multiple storage devices, i.e., one or more storage devices.

The input interface 21 accepts input from an administrator of the computer system 2 (e.g., an employee of the management organization) and outputs an input signal corresponding to the accepted input to the processor 26. The communication module 22 is connected to the communication network CN and communicates with each of the terminals 1-1, ..., 1-N. This communication may be wired or wireless.

The storage device 23 is, for example, a storage, and stores programs and various data to be read and executed by the processor 26. The memory 24 temporarily holds the data and programs. The memory 24 is a volatile memory, for example, a RAM (Random Access Memory).

The output interface 25 can be connected to an external device and can output a signal to the external device. The processor 26 loads a program from the storage device 23 into the memory 24 and executes a series of instructions contained in the program to perform various processes described below.

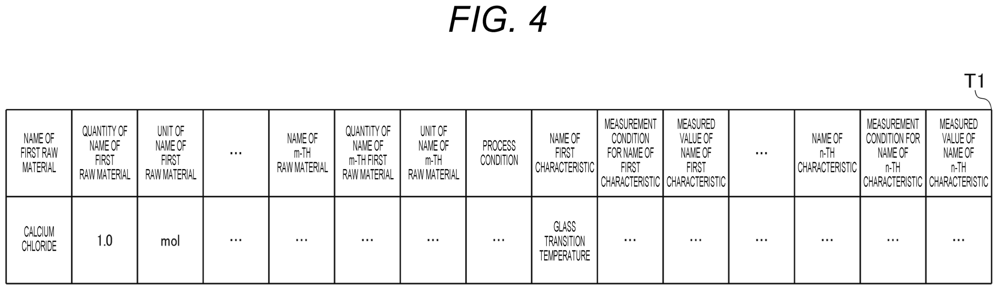

FIG. 4 is an example of a table stored in a storage device of a computer system. As shown in FIG. 4, table T1 stores data for each chemical composition. For example, in table T1, one record includes the name of the first raw material constituting one chemical composition, its amount, its unit, ..., the name of the m-th raw material (m is a natural number), its amount, and its unit. Furthermore, one record includes a set of the process conditions for making the chemical composition, the first characteristic name of the chemical composition, its measurement conditions, its measured value, ..., the nth property name (n is a natural number) of the chemical composition, its measurement conditions, and its measured value. Some chemical compositions are composed of one raw material, while others are composed of multiple raw materials. Even when a chemical composition is composed of multiple raw materials, the number of raw materials constituting the chemical composition may vary.

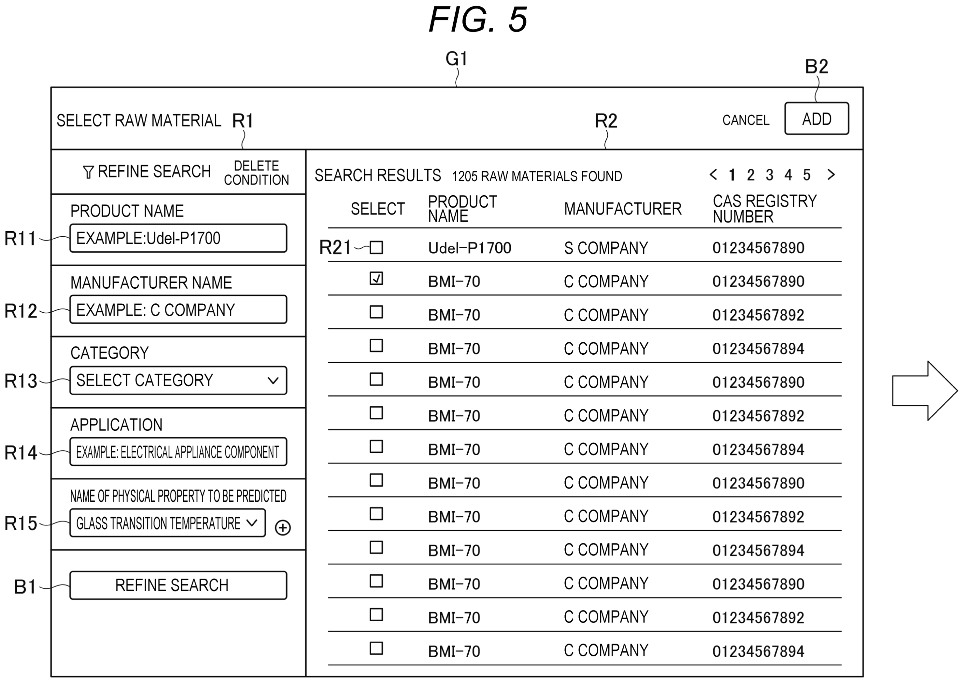

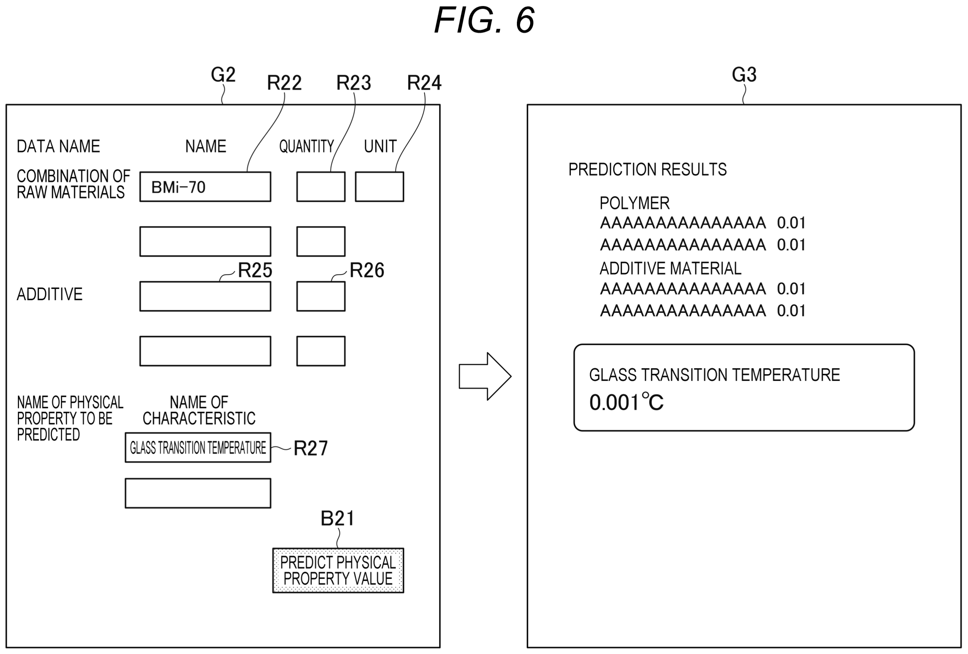

Next, screen transitions in the terminal of the first embodiment will be described with reference to Figs. 5 and 6. Fig. 5 is a diagram showing an example of a screen for selecting raw materials displayed on the terminal of the first embodiment. Fig. 6 is an example of a screen subsequent to that shown in FIG. 5. As shown in Fig. 5, the screen G1 has a screen region R1 for setting search conditions for raw material products and a screen region R2 for displaying the search results. The screen region R1 has a text box R11 for inputting the current product name, a manufacturer name input R12, a select box R13 for selecting a category (specific examples include acrylic resin, titanium oxide, zirconia, etc.), a text box R14 for inputting an application (specific examples include adhesives, etc.), a select box R15 for selecting the predicted object property name, and a "narrow down" button B1. When narrowing down the results by setting conditions, the "narrow down" button B1 is pressed. When the "narrow down" button B1 is pressed, the search results are displayed in the screen region R2. A list of product candidates found by the search is displayed in screen area R2, and for each product found, a selection box R21, the product name, manufacturer name, and CAS registration number are displayed.

A database has been constructed in the storage device 23, and a product table, a use master table, a category master table, and a physical property table are stored. In the product table, for example, a product ID, which is an example of product identification information that identifies a product, is associated with a product name, a manufacturer ID, a CAS registration number, a use ID that identifies a use, and a category ID that identifies a category. In the use master table, the use ID and the use name are stored in association with each other. In the physical property table, the product ID and the physical property values of the product are stored in association with each other.

The following describes the processing of processor 26 up to displaying the product candidate list when "Refine" button B1 on screen G1 in Figure 5 is pressed. Processor 26 extracts at least one raw material candidate based on at least one of the raw material product name, raw material manufacturer name, raw material category, and raw material use received from the user, outputs information for displaying the extracted raw material candidate in a selectable manner, and transmits it to terminal 1. As a result, the product candidate list hit by the search is displayed in screen area R2 of screen G1 of terminal 1 that received this information.

When the Add button B2 is pressed with at least one selection box R21 in screen region R2 of screen G1 selected, the screen transitions to screen G2 of FIG. 6. In screen G2 of FIG. 6, a text box R22 in which the name of a raw material can be input, a text box R23 in which the amount can be input, and a text box R24 in which the unit can be input are displayed, allowing the name, amount, and unit to be input for each raw material. Also a text box R25 in which the name of an additive can be input, and a text box R26 in which the amount can be input are displayed, allowing the name and amount of the additive to be input. Also a text box R27 in which the name of the predicted target physical property can be input is displayed, allowing the name of the predicted target physical property to be input.

In this example, the name of the raw material corresponding to the selection box R21 selected in the screen region R2 of the screen G1 is displayed in a text box R22. Also, as another example, the name of the predicted target property selected in the selection box R15 in the screen region R1 of the screen G1 is displayed in a text box R27.

Note that in screen G2 in FIG. 6, further the name of the device to be used for processing so that it can be input may be displayed, or the processing conditions so that it can be input may be displayed. In this case, the device name and/or processing conditions may be added to the input of the machine learning model.

When at least one raw material, its amount and unit, the name of the target physical property to be predicted, and additives are input as necessary, and then the Physical Property Prediction button B12 is pressed, the screen transitions to screen G3.
Screen G3 displays the prediction result of physical property values of input name of the target physical property to be predicted for the composition produced from the input combination of raw materials and the quantities of each, and the combination of additives and the quantities of each. In the example of screen G3 in Figure 6, the predicted value of the glass transition temperature is displayed as an example.

### <Method for predicting the physical property value of prediction target physical property name>

Next, regarding the method for predicting the physical property value of the prediction target physical property name, first, an outline of the process will be described, and then, a specific processing method will be described. The storage device 13 stores a trained machine learning model. Specifically, for example, the storage device 13 stores a machine learning model constructed for each prediction target property name. In some cases, a prediction target property name may be added and a machine learning model may be constructed for the added prediction target property name. The processor may construct a machine learning model by learning from scratch, or may add or update teacher data and retrain the stored machine learning model. Here, the machine learning model is, for example, a model trained by a learning data set that takes as input a chemical fingerprint obtained by converting a combination of the names of raw materials constituting a chemical composition and the amount of each of the raw materials, and takes as outputs as a physical property value of the prediction target physical property name. In this case, when a user inputs a combination of the names of raw materials and the amount of each of the raw materials, processor 26 may, for example, convert each numerical value of the obtained fingerprints, that is obtained by converting the input raw materials, into a corresponding chemical fingerprint by weighting the numerical values of each of the fingerprints with the amount of each raw material. In the step of converting into a fingerprint, when a raw material is input by a user, processor 26 may, for example, convert the raw material into a one-line character string (e.g., CC1=CC2) using characters and symbols in the notation of Simplified Molecular Input Line Entry System (SMILES) (or read it as a character string), and further convert the converted character string into a chemical fingerprint represented by a numerical string.

On the other hand, when only one raw material is input, processor 26 may convert the raw material into a chemical fingerprint.

Alternatively, for example, the machine learning model may be a model trained from a learning data set that includes, as input, chemical fingerprints converted from each of the raw materials constituting the chemical composition and the amounts of each of the raw materials, and as output, a physical property value of a predicted physical property name as output. In this case, when a user inputs a combination of raw material names and the amounts of each of the raw materials, processor 26 may convert each of the input raw materials into a corresponding chemical fingerprint.

As yet another alternative, for example, the machine learning model may be a model trained from a learning data set that includes, as input, each of information identifying the raw materials that make up the chemical composition (e.g., index, name, CAS registry number, etc.) and the amount of each of the raw materials, and as output, the physical property value of the predicted physical property name.

Processor 26 acquires the combination of raw material names and the amounts of each of the raw materials received from the user, converts the combination of raw material names and the amounts of each of the raw materials into a chemical fingerprint, and inputs the obtained chemical fingerprint into a trained machine learning model stored in storage device 23 to acquire a predicted value of the physical property value of the predicted target physical property name. More specifically, for example, processor 26 acquires a predicted value of the physical property value of the predicted target physical property name by inputting the chemical fingerprint into a machine learning model corresponding to the predicted target physical property name received from the user.

In addition, a plurality of fingerprints may be input to the machine learning model, and the number of fingerprints input is not limited to two, but may be three or more. Specifically, the machine learning model may be a model that is trained by inputting a plurality of fingerprints and outputting a predicted value of the physical property value of the predicted target physical property name. As an example, the input of the second machine learning model may further include a second chemical fingerprint obtained by converting the chemical fingerprints corresponding to each additive by, for example, weighting the amount of each additive. In this case, processor 26 may obtain a predicted value of the physical property value of the predicted target physical property name by inputting at least one of the chemical fingerprints and at least one of the second chemical fingerprints to the trained second machine learning model. For example, when distinguishing additives by type, in addition to the second chemical fingerprint, there may be a third fingerprint or later. In addition, there may be four or more fingerprints in total, such as fingerprints of two or more raw materials (e.g., polymers) with different roles and fingerprints of two or more additives with different roles, and by inputting these four or more fingerprints into a trained second machine learning model, a predicted value of the physical property value of the physical property name to be predicted may be obtained.

Although the above describes an example of conversion to a chemical fingerprint, the present invention is not limited to this. The processor 26 may convert the chemical formula of at least one raw material (and/or at least one additive) into a character string in the notation of SMILES (hereinafter, also referred to as a SMILES character string), input the converted SMILES character string or a group of numerical values (e.g., a vector), converted from each SMILES character string, into a machine learning model (e.g., a machine learning model of a natural language processing system when a character string is input, or, for example, a neural network when a group of numerical values is input), and output a predicted value of the physical property value of the predicted physical property name. Here, as a method of conversion to a vector, for example, a technique called Smiles2vec described in Non-Patent Document 1 may be used to convert the SMILES character string into a vector. This Smiles2vec technique uses layers such as LSTM (Long short-term memory) and GRU (Gated recurrent unit) based on the idea of RNN (Recurrent Network: Recurrent Neural Network). Here, the machine learning model is a model trained using training data in which a SMILES character string or a group of numerical values based on a SMILES character string (e.g., the above-mentioned converted vector) is input, and a predicted value of the physical property value of the predicted physical property name is output. This group of values may be a vector (e.g., a one-dimensional array) or a matrix (multidimensional array).

Alternatively, the processor 26 may convert the chemical formula of at least one raw material (and/or at least one additive) into chemical graph structure data (e.g., including an adjacency matrix) that represents a chemical graph structure, input the converted information to a machine learning model (e.g., a machine learning model including a graph neural network in the front stage and a fully connected neural network in the rear stage), and output a predicted value of the physical property value of the predicted target physical property name. For example, in the case of an adjacency matrix, it is converted into a vector using a graph convolutional neural network, and the converted vector is input to a fully connected neural network. On the other hand, for example, the chemical graph structure data may be converted into a vector (e.g., converted into 0 or 1 depending on whether a specific rule is satisfied such as a fingerprint or MACCS Keys), and the vector may be input to a (e.g., fully connected) neural network.

Here, the machine learning model is a model trained by learning data in which chemical graph structure data or a group of numerical values (e.g., vectors) converted based on the chemical graph structure data is input, and a predicted value of the physical property name of the prediction target is output. This group of values may be a vector (one-dimensional array) or a matrix (multidimensional array).

Here, as a method of converting into a numerical sequence (e.g., a vector) representing a graph structure, for example, a fingerprint may be obtained by converting into 0 or 1 depending on whether a specific rule such as MACCS Keys is satisfied. On the other hand, in the case of an adjacency matrix representing a graph structure, this adjacency matrix may be input into a graph convolution network, and the vector output from the final layer of the graph convolution network may be used as the converted vector. Here, to convert from a graph structure to a vector, for example, one or more convolution processes may be performed, followed by conversion using the Readout function described in Non-Patent Document 2 (https://docs.dgl.ai/en/0.8.x/guide/training-graph.html). In this case, for example, a specific feature of the graph (e.g., the number of C-C bonds, etc.) may be counted and converted into a vector. Then, after converting from the graph structure to a vector, an operation of creating a vector by weighting and averaging each according to the amount may be created in a neural network (e.g., a fully connected neural network).

Here we explain a specific example of a process for creating a vector that is a weighted average of compounds a and b according to their amounts. Let G_a be the adjacency matrix of compound a. This adjacency matrix is, for example, a square matrix in which atoms are treated as nodes and bonds as edges, and the presence or absence of an edge (1 or 0) between each node is stored. H_a is, for example, a vector obtained by performing one or more convolution processes on the adjacency matrix of compound a, followed by executing the Readout function.

Similarly, the adjacency matrix for compound b is G_b, and H_b is the vector obtained by performing one or more convolution processes on the adjacency matrix of compound b and then executing the Readout function.

The vector H_total, which is the weighted average of compounds a and b according to their amounts (hereinafter referred to as the weighted average vector), can be expressed, for example, by the following formula. H_total = H_a × amount of compound a + H_b × amount of compound b

The weighted average vector H_total is input to the subsequent layer (e.g., the fully connected layer) of this machine learning model, and the predicted values of the physical properties are output from the output layer of the machine learning model. By using such a machine learning model, it is possible to improve the prediction accuracy of the physical properties from the chemical structures in various expression and their amounts formats.

In this way, the input of the second machine learning model may further include a set of numerical values obtained by conversion using the second chemical fingerprint, the second SMILES character string, or the second chemical graph structure corresponding to each of the additives and the amount of each of the additives, and processor 26 may obtain a predicted value of the physical property value of the predicted target physical property name by inputting the set of numerical values based on either the chemical fingerprint, the SMILES character string, or the chemical graph structure corresponding to each of the raw materials, and the set of numerical values based on either the second chemical fingerprint, the second SMILES character string, or the second chemical graph structure corresponding to each of the additives to the second machine learning model.

Alternatively, the input of the first machine learning model may further include a second SMILES string or a second chemical graph structure corresponding to each of the additives, and the processor 26 may obtain a predicted value of the physical property value of the predicted target physical property name by inputting the SMILES string or the chemical graph structure corresponding to each of the raw materials and the amount of each of the raw materials, and the second SMILES string or the second chemical graph structure corresponding to each of the additives and the amount of each of the additives into the first machine learning model.

The specific process will be described in detail below. For example, a database is constructed in the storage device 23, and data on the working examples collected from patent documents (e.g., raw material names and structures, physical properties, amounts of each raw material used, process conditions, characteristic values, etc.) and raw material data used for similar or the same purposes as those of the working examples (e.g., raw material names, structure data, property information described in catalogs, available information such as CAS numbers, etc.) are stored.

Next, an example of a method for constructing a machine learning model will be described.

(Step S10) For example, the collected data of a working example is processed into data for machine learning. Specifically, the units of the example data are aligned and converted into a chemical fingerprint. If a working example is a mixture, the mixture is converted into a chemical fingerprint by taking a weighted average based on the amounts of raw materials contained in the mixture. Any outliers are excluded.

(Step S20) Next, a machine learning model is constructed. The machine learning model may be, for example, a random forest, a Gaussian process regression, a neural network model, or another Bayesian model.

### <Machine learning model construction process>

A first machine learning model (or a second machine learning model) is constructed for each prediction target physical property name by executing the following machine learning model construction process, for example, for each prediction target physical property name. Here, as an example of the first machine learning model, the machine learning model is a model in which parameters are adjusted so that the output can be predicted from the input using a learning data set in which a group of numerical values obtained by converting the chemical fingerprints corresponding to each raw material and the amounts of each raw material is input, and the physical property values of the physical property names is output.

Although a model that learns for one physical property will be described as an example here, a model that learns for multiple physical properties simultaneously may also be used. For example, in the case of a neural network, multiple physical properties may be learned simultaneously using multitask learning. This machine learning model also includes regression analysis methods. These regression analysis methods include Bayesian linear regression and partial least squares regression (PLS). For example, as an example of regression analysis, a regression analysis method called partial least squares regression (PLS) may be used to target multiple physical properties. Since input variables and output variables are learned simultaneously, multiple physical properties may be estimated. PCR (Principal Component Regression) may be used, or one or more physical properties may be predicted using a Bayesian model (for example, Bayesian linear regression or hierarchical Bayesian model).

(Step S21) In order to improve generalization performance, working examples included in the same patent document are grouped together, and all working examples are divided into K sets of training and validation data (K is a natural number) using Group-K fold.

(Step S22) The optimal combination of hyperparameters for the selected machine learning model is verified using the validation data. Each combination of hyperparameters is trained using K sets of training data, and accuracy is calculated using the validation data. The average of the K accuracies is set as the accuracy of each selected hyperparameter. The combination of hyperparameters with the highest accuracy in the validation data is selected.

(Step S23) All data is trained using the combination of hyperparameters with the highest accuracy, and the machine learning model is saved.

### <Machine learning model estimation process>

Processor 26 obtains a weighted average of the chemical fingerprints by using, for example, a combination of information identifying raw materials entered by the user (e.g., index, name, CAS registration number, etc.) and the amount of each of the raw materials in a manner similar to that described above. Processor 26 then obtains a predicted value of the physical property value of the predicted target physical property name by inputting the obtained chemical fingerprints into the machine learning model saved in step S23 above.

As described above, the information processing system S according to the first embodiment comprises least one processor 26 that acquires a combination of information (e.g., index, name, CAS registration number, etc.) identifying each raw material received from a user and the amount of each raw material, and acquires a predicted value of the physical property value of the predicted target physical property name by inputting the chemical fingerprint, SMILES character string, or chemical graph structure data corresponding to each raw material and the amount of each raw material to a first machine learning model, or by inputting a set of numerical values based on the chemical fingerprint, SMILES character string, or chemical graph structure data corresponding to each raw material and the amount of each raw material to a second machine learning model. Here, the first machine learning model is a model in which the parameters are adjusted so that the output can be predicted from the input using a learning data set in which the chemical fingerprint, SMILES character string, or chemical graph structure data corresponding to each raw material and the amount of each raw material are input, and the physical property value of the predicted target physical property name is output. The second machine learning model is a model in which parameters are adjusted so that the output can be predicted from the input using a learning data set in which a group of numerical values obtained by converting at least one of a chemical fingerprint, a SMILES string, or chemical graph structure data, or a product name or substance name corresponding to each raw material and the amount of each of the raw materials is input, and the physical property value of the physical property name to be predicted is output.

According to this configuration, a user can obtain a predicted physical property value of the predicted physical property name of the composition obtained by combining the raw materials by inputting a combination of raw materials and the amounts of each of the raw materials. This makes it easy to obtain the physical property values of the composition obtained by combining the raw materials.

### <Second Embodiment>

Next, a second embodiment will be described. In the second embodiment, an information processing system that solves the second problem described above will be described. In the second embodiment, when a desired range of physical property values is input, candidate combinations of raw materials are output. Since the hardware configuration of the information processing system according to the second embodiment is similar to that of the information processing system according to the first embodiment, its description will be omitted.

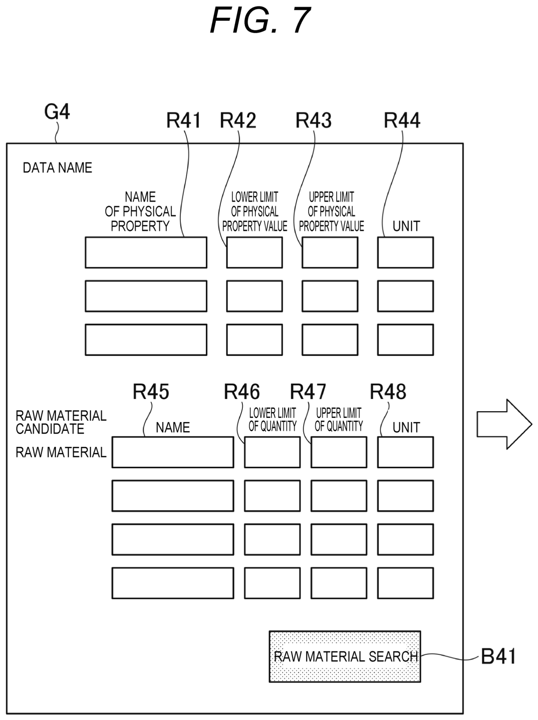

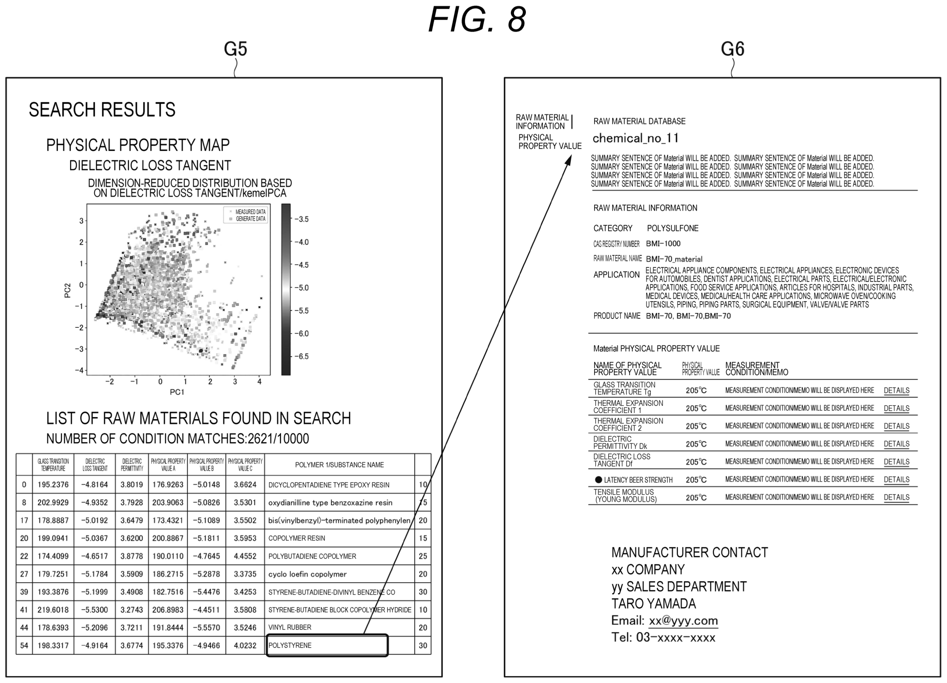

Fig. 7 is a diagram showing an example of a screen for selecting raw materials displayed on a terminal according to the second embodiment. Fig. 8 is an example of a screen subsequent to Fig. 7.

In screen G4 of Figure 7, a text box R41 in which the desired physical property name can be input, a text box R42 in which the desired lower limit of the physical property value can be input, a text box R43 in which the desired upper limit of the physical property value can be input, and a text box R44 in which the unit can be input are displayed.

Also, on screen G4 in FIG. 7, for the raw material that is to be used at a minimum to obtain the desired range of physical property values, a text box R45 in which the name of the raw material can be input, a text box R46 in which the lower limit of the amount of the raw material can be input, a text box R47 in which the upper limit of the amount of the raw material can be input, and a text box R48 in which the unit of the amount can be input are displayed. When the raw material search button B41 is pressed on screen G4 in FIG. 7, screen transitions to screen G5 in FIG. 8.

Screen G5 in FIG. 8 is a screen that displays the results of the raw material search. For example, a physical property map is displayed in which the physical property values of each raw material are plotted two-dimensionally for the physical property name specified on screen G4 in FIG. 7. Screen G5 in FIG. 8 displays the raw material list obtained as a result of the raw material search. The raw material list includes various physical property values, combinations of substance names, and the amounts of each of the substance names. For simplicity, FIG. 8 shows the case where there is one substance name.

For each substance name, a link is provided to a web page containing information about that substance. The page containing the information about that substance includes, for example, a description of the substance, its physical properties, and contact information for the manufacturer (or its agent). When a specific operation (e.g., left-click) is performed on a specific substance name (here, for example, "polystyrene"), the screen transitions to screen G6 which contains information about the specific substance name (here, for example, "polystyrene"). Screen G6 includes a description of the specific substance name (here, for example, "polystyrene"), its physical properties, and contact information for the manufacturer (or its agent).

As in the first embodiment, the storage device 23 in the second embodiment stores a trained machine learning model. Specifically, for example, the storage device 23 stores a machine learning model constructed for each physical property name. Here, the machine learning model is, for example, a model trained by a learning data set that takes as input a chemical fingerprint obtained by converting a combination of raw materials and the amount of each of the raw materials, and takes as output the physical property value of the physical property name.

Next, an example of a method for constructing a machine learning model will be described.

(Step S110) As in step S10 of the first embodiment, for example, collected working example data is processed into data for machine learning. Specifically, the units of the working example data are aligned and converted into a chemical fingerprint. If the working example is a mixture, the mixture is converted into a chemical fingerprint by taking a weighted average based on the amounts of raw materials contained in the mixture. Any outliers are excluded.

(Step S120) Next, a machine learning model is constructed in the same manner as in step S20 of the first embodiment. The machine learning model is, for example, a random forest, a Gaussian process regression, a neural network model, or another Bayesian model.

### <Machine learning model construction process>

As an example, the following machine learning model construction process is executed for each predicted target property name, and a machine learning model (specifically, for example, a first machine learning model or a second machine learning model) is constructed for each predicted target property name.

(Step S121) As in step S21 in the first embodiment, in order to improve generalization performance, working examples included in the same patent document are grouped together, and all working examples are divided into K sets of training and validation data (K is a natural number) using Group-K fold.

(Step S122) As in step S22 of the first embodiment, the optimal combination of hyperparameters for the selected machine learning model is verified using validation data. Each hyperparameter combination is trained with K sets of training data, and accuracy is calculated using the validation data. The average of the K accuracies is set to the accuracy of each selected hyperparameter. The hyperparameter combination with the highest accuracy in the validation data is selected.

(Step S123) As in step S23 of the first embodiment, all data is trained using the hyperparameter combination with the highest accuracy, and the machine learning model is saved.

### <Search process>

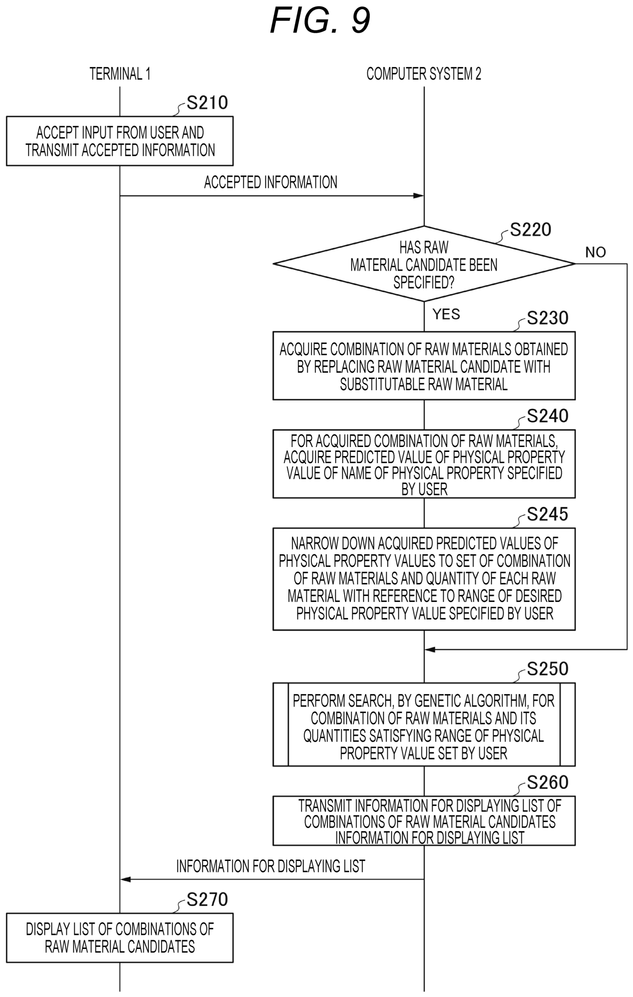

FIG. 9 is a flowchart showing an example of the flow of a search process according to the second embodiment.

(Step S210) The terminal 1 accepts input from the user, for example, on screen G4 in Fig. 7. The information accepted from the user here is the name of the physical property, the range of values of the physical property name, and the unit of the value. In addition, the information accepted from the user is the candidate raw materials, the range of amounts of the raw materials, and the unit of the amounts. The terminal 1 transmits the information accepted from the user to the computer system 2.

(Step S220) When processor 26 receives the information transmitted in step S210, it determines whether or not a candidate raw material has been designated by the user (i.e., whether or not the received information includes a candidate raw material).

(Step S230) When a candidate raw material is designated by the user (i.e., when the received information includes a candidate raw material), the processor 26 obtains, for example, substitutable raw materials for the candidate raw material designated by the user in FIG. 7 from the catalog, and obtains a raw material combination obtained by replacing the candidate raw material with the obtained substitutable raw material.

(Step S240) Processor 26 may convert each of the raw materials included in the raw material combination obtained in step S230 into a chemical fingerprint, and input the obtained chemical fingerprints (and the amounts of each raw material) into the trained machine learning model obtained in step S120, thereby obtaining a predicted value of the physical property value of the physical property name specified by the user in Fig. 7, for example. Here, the initial value of the amount of each raw material may be set in advance or may be specified by the user.

(Step S245) The processor 26 narrows down the obtained predicted values of the physical properties to combinations of raw materials and amounts of each of the raw materials that satisfy the desired range of the physical properties designated by the user.

(Step S250) Then, processor 26 uses a genetic algorithm to search for a combination of raw materials and their amounts that satisfy the physical property value range set by the user in Fig. 7. As one example, processor 26 may use a genetic algorithm to search for a combination of substitutable raw materials, whose physical property value set by the user has the highest or lowest or falls within a specific range (e.g., 100 to 200), and their amounts.

Here, the substitutable raw materials may be obtained from a catalog stored in the storage device 23, or the substitutable raw materials may be obtained from catalog information on the WEB via the Internet.

### <Specific example of search method using genetic algorithm>

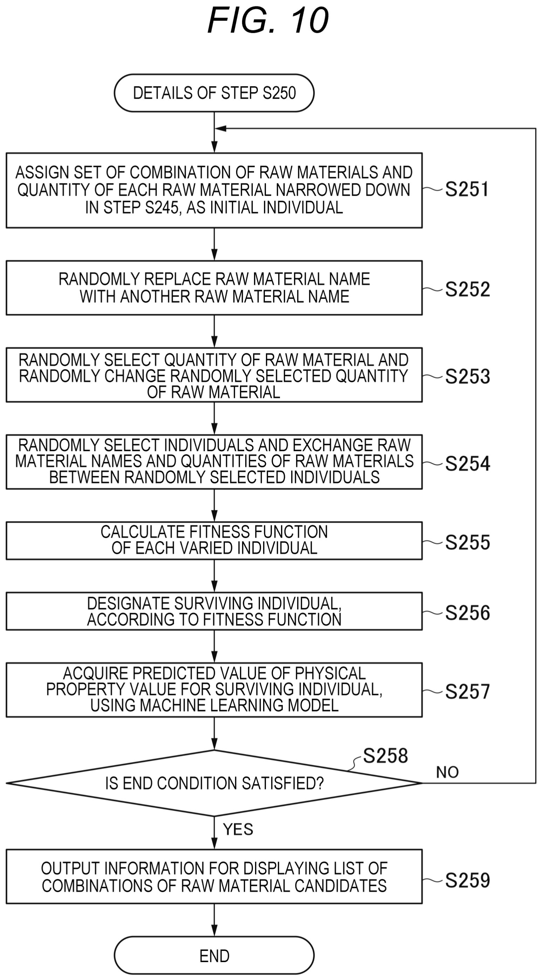

A specific example of the search method using the genetic algorithm in step S250 will now be described. Fig. 10 is a flow chart showing an example of the flow of the search process using the genetic algorithm in step S250 in Fig. 9.

### (Step S251: Initial Individual of Genetic Algorithm)

The processor 26 may set, for example, a combination of raw materials narrowed down in step S245 and the amount of each raw material as an initial individual. Here, an individual is specified by a combination of information for identifying raw materials (e.g., ingredient names) and the amount of each raw material.

Alternatively, the processor 26 may randomly select an initial individual for the genetic algorithm from among the combinations of raw material names and the amounts of each raw material described in the examples of the patent document. Here, the combinations of information identifying raw materials (e.g., raw material names) and the amounts of each raw material described in the examples of the patent document may be stored in the storage device 23, and in that case, the processor 26 may obtain the combinations of information identifying raw materials (e.g., raw material names) and the amounts of each raw material from the storage device 23.

(Step S252: Mutation) The processor 26 randomly replaces information identifying a raw material (e.g., raw material name) with information identifying another raw material (e.g., raw material name).

(Step S253: Mutation 2) Processor 26 randomly selects the amount of raw material, and randomly changes the amount of the randomly selected raw material.

(Step S254: Crossover) The processor 26 randomly selects an individual by regarding a pair of information for identifying a raw material (e.g., raw material name) and the amount of the raw material as one individual, and exchanges the information for identifying a raw material (e.g., raw material name) and the amount of the raw material between the randomly selected individuals. At this time, the processor 26 adjusts the amount ratio so that the total becomes 100, for example.

(Step S255) The processor 26 calculates a fitness function for each mutated individual. The fitness function may be, for example, a difference between a predicted value of a physical property value and a representative value (e.g., median, average, maximum, or minimum value) of a physical property value range set by a user, or may be something like a likelihood function if the machine learning model is a model capable of outputting a probability distribution, the predicted value of a physical property value being obtained by using a combination of information identifying the raw material (e.g., raw material name) and the amount of each raw material, for example, by converting a chemical fingerprint corresponding to each raw material into a chemical fingerprint by weighting the amount of each raw material and by inputting the converted chemical fingerprint into a trained machine learning model.

Here, as an example, the converted chemical fingerprint is input to the trained machine learning model (the second machine learning model described above) to obtain a predicted value of the physical property value, but the present invention is not limited to this. The SMILES character string or chemical graph structure data corresponding to each raw material and the amount of each raw material may be input into the first machine learning model described above to obtain a predicted value of the physical property value.

In addition, as an example, it has been described here that the chemical fingerprints corresponding to the respective raw materials and the converted chemical fingerprints, which are a set of numerical values based on the amounts of the respective raw materials, are input to the trained machine learning model (the above-mentioned second machine learning model) to obtain predicted values of the physical properties, but this is not limited to this. It is also possible to input the SMILES character strings or chemical graph structure data corresponding to the respective raw materials and a set of numerical values based on the amounts of the respective raw materials to the second machine learning model to obtain predicted values of the physical properties.

(Step S256) The processor 26 determines surviving individuals (also called surviving individuals) according to the fitness function. At that time, the processor 26 may execute a process of imposing a penalty on the fitness function if it falls outside a range specified by the user (for example, a range including raw materials specified by the user and/or a physical property value specified by the user is in the range of ○ to ○, etc.). This allows for efficient search.

(Step S257) Processor 26 converts the chemical fingerprints corresponding to the raw materials into a chemical fingerprint by, for example, taking a weighted average of the amounts of the raw materials using the combination of raw materials and the amounts of the raw materials corresponding to the surviving individuals, and inputs the converted chemical fingerprints into the trained machine learning model to obtain a predicted value of the physical property value. In this case, processor 26 may, for example, convert each of the raw materials corresponding to the surviving individuals into a fingerprint, and convert the numerical values of each of the obtained fingerprints into a chemical fingerprint by taking a weighted average of the respective amounts.

(Step S258) Then, the processor 26 determines whether or not a termination condition based on the physical property value range specified by the user is satisfied. If the termination condition is not satisfied, the processor 26 returns to step S251 and repeats the above-mentioned process. In this way, the processor 26 repeats the above-mentioned process until the termination condition is satisfied. The termination condition may be a condition that an optimal physical property value (also called an optimal solution) that the predicted physical property value acquired in step S257 satisfies the physical property value range specified by the user is found. Or a condition that a set number or more combinations of candidate raw materials that the predicted physical property value acquired in step S257 satisfies the physical property value range specified by the user are found. Here, the combination of candidate raw materials may consist of one candidate raw material or may consist of multiple candidate raw materials.

(Step S258) If the predetermined termination condition is met in step S257, the processor 26 outputs, for example, information for displaying a list (for example, a ranking display) of combinations of candidate raw materials.

(Step S260) Returning to FIG. 9, the processor 26 transmits, for example, information for displaying a list of combinations of candidate raw materials to the terminal 1.

(Step S270) When terminal 1 receives information for displaying the list, it uses this information to display a list of combinations of candidate raw materials. As a result, as shown on screen G5 of FIG. 8, terminal 1 displays a list (e.g., a ranking display) of combinations of candidate raw materials. When outputting information for displaying the list, processor 26 may, for example, output information such that a link is set to the raw material name. As a result, as shown on screen G5 of FIG. 8, a link is set to the raw material name when the list is displayed on terminal 1. As a result, by clicking on the link set for a raw material by a user, it is possible to transition to a web page (see, for example, screen G6) of product information for that raw material (e.g., a catalog, how to contact the manufacturer, etc.) as shown in FIG. 8.

As described above, the information processing system according to the second embodiment includes at least one processor. The processor regards a pair of a combination of raw materials and the amounts of each of the raw materials as a parent population, creates a child population of the next generation according to a genetic algorithm, calculates a fitness function value using the combination of raw materials and the amounts of each of the raw materials indicated by the created child population of the next generation, repeats the process of creating a child population of the next generation until a termination condition based on a physical property value range specified by the user is satisfied, and when the termination condition is satisfied, outputs information including the combination of raw materials and the amounts of each of the raw materials indicated by each of the child populations of the next generation lastly created.

According to this configuration, the user can obtain a combination of raw materials and the amount of each of the raw materials that satisfy the physical property range specified by the user.

Processor 26 may also output predicted values of the physical property value of the desired physical property name exhibited by each of the lastly created offspring populations of the next generation. This allows the user to grasp the predicted values of the physical property value of the desired physical property name.

In the above step S257, the combination of raw materials and the amount of each of the raw materials corresponding to the living individual are used to convert the chemical fingerprints corresponding to the raw materials into chemical fingerprints by, for example, weighting the amount of each of the raw materials, and the converted chemical fingerprints are input to the trained machine learning model to obtain the predicted value of the physical property value, but this is not limited to this.

For example, the processor may obtain at least one set of desired physical property ranges input by the user for the desired physical property name, and input the chemical fingerprints, SMILES character strings, or chemical graph structure data corresponding to each of the raw materials included in the initial combination of raw materials, and the amount of each of the raw materials to the first machine learning model, or input a group of numerical values based on the chemical fingerprints corresponding to each of the raw materials (however, a specific example using this chemical fingerprint corresponds to step S257), SMILES character strings, or chemical graph structure data, and the amount of each of the raw materials to the second machine learning model to obtain the predicted value of the physical property value of the physical property name, as in the first embodiment.

Here, the first machine learning model is a model in which the parameters are adjusted so that the output can be predicted from the input using a learning data set in which a chemical fingerprint, a SMILES character string, or chemical graph structure data corresponding to each raw material, and the amount of each raw material are input, and the physical property value of the prediction target physical property name is output.

The second machine learning model is a model in which the parameters are adjusted so that the output can be predicted from the input using a learning data set in which a group of numerical values obtained by conversion using the chemical fingerprint, a SMILES character string, or chemical graph structure data corresponding to each raw material, and the amount of each raw material is input, the physical property value of the physical property name is output.

The processor, for example, narrows down the obtained predicted values of the physical properties to combinations of raw materials and sets of the amounts of each of the raw materials that satisfy the desired range of physical properties specified by the user, and regards the narrowed down combinations of raw materials and sets of the amounts of each of the raw materials as a parent population, and creates the next generation of child populations according to a genetic algorithm.

This allows the narrowed-down combinations of raw materials and the amounts of each of the raw materials to be treated as a parent population and a genetic algorithm to be run, thereby making it possible to obtain, in a shorter time, combinations of raw materials and the amounts of each of the raw materials that satisfy the physical property range specified by the user.

The storage device 23 may store a machine learning model constructed for each physical property name. In this way, the first machine learning model or the second machine learning model may be constructed for each physical property name. In this case, the processor 26 may obtain a predicted value of the physical property value of the prediction target physical property name by inputting the converted chemical fingerprint into a machine learning model corresponding to the physical property name received from the user. According to this configuration, a predicted value of the physical property value can be obtained for each physical property name, so that a combination of raw materials that satisfies the physical property value range specified by the user and the amount of each of the raw materials can be obtained for the physical property name desired by the user.

Furthermore, when a user selects one of the outputted raw materials, the processor 26 may output contact information for the sales company that sells the raw material. This configuration allows the user to easily obtain raw materials, and enables the administrator of the computer system 2 to earn advertising revenue or affiliate revenue from the sales company that sells the raw material.

When a user selects one of the outputted raw materials, the processor 26 may output detailed information about the raw material. This configuration allows the user to easily obtain detailed information about raw materials that satisfy the physical property value range specified by the user.

### <Third Embodiment>

Next, a third embodiment will be described. In the third embodiment, an information processing system that solves the third problem will be described. In the third embodiment, working examples that use the raw material name (or product name) input by the user and/or working examples that do not use the input raw material among working examples that show physical property values similar to those of the extracted working example are extracted from the data of working examples of patent documents stored in a storage device, and a list of the applicants (companies that use the raw material) is output. This makes it possible to obtain the names of companies that are likely to use products with the raw material name (or product name) input by the user.

In the third embodiment, a list of raw materials included in the working examples that show similar physical property values may be output, allowing the user to obtain a list of raw material names that are likely to be substituted, which can be used for sales or promotion of the raw material name (or product name) entered by the user.

The storage device 23 stores, for example, data on working examples included in patent documents (such as raw material names and/or product names, structures, physical properties, amounts of each raw material used, process conditions, physical property values, etc.), and in addition to the above, the filing date, the name of the applicant, and the names of manufacturers of the raw materials described in the patent documents. This information may be stored in a database constructed in the storage device 23.

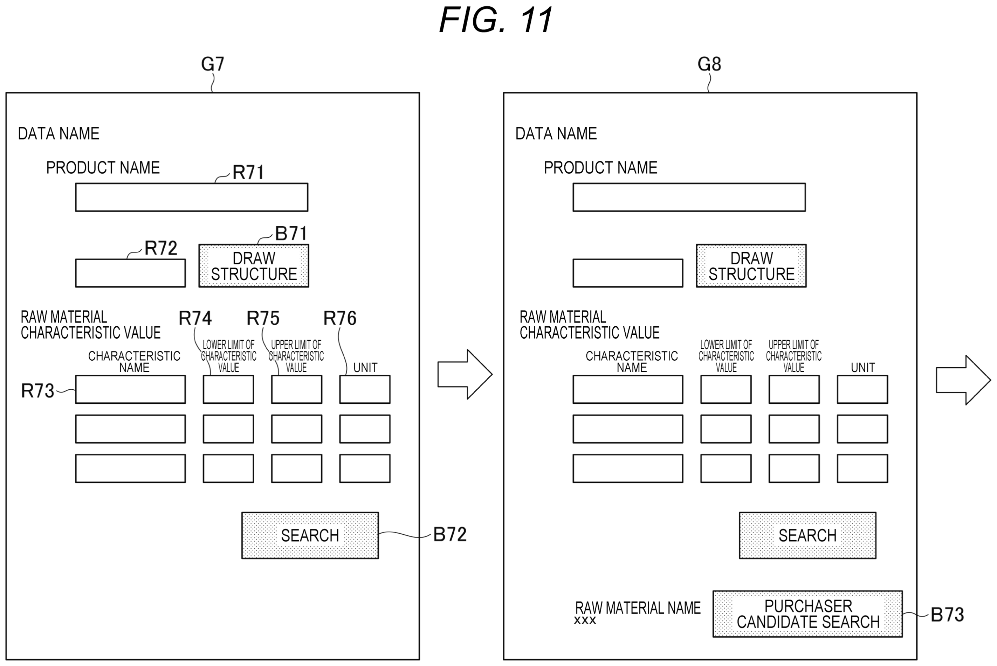

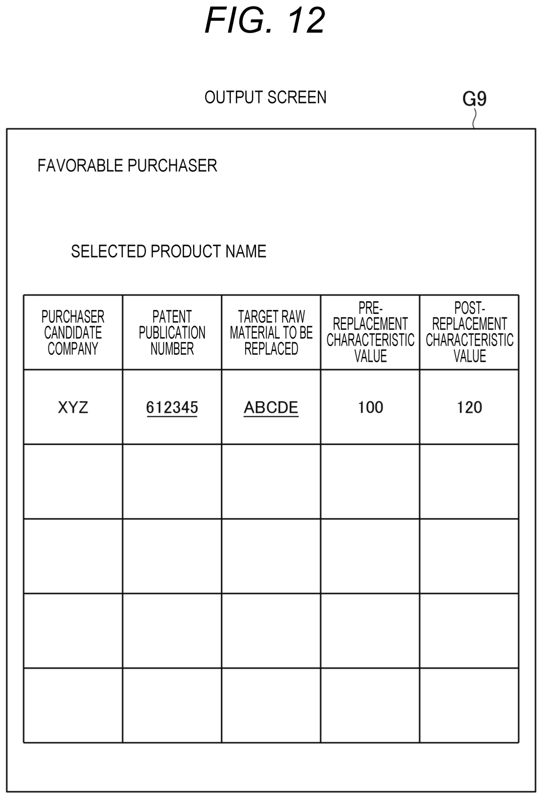

FIG. 11 is a diagram showing an example of screen transitions in a terminal of the third embodiment. FIG. 12 is an example of a screen subsequent to FIG. 11.

In the screen G7 of FIG. 11, a text box R71 is shown for the user to input a product name. For example, when the user inputs part of a product name in the text box R71, a list of candidates with partial matches may be displayed with check boxes, and the user may select a candidate with the check box. In case the user cannot think of a product name, the screen G7 may be provided with a text box R72 for inputting the chemical formula of the raw materials contained in the product. Similarly, in case the user cannot think of a product name, a structure drawing button B71 for drawing the structure of the raw materials contained in the product may be provided. When the structure drawing button B71 is pressed, another screen is displayed as a pop-up, and the user can draw the structure.

The characteristic values of each raw material are stored in advance in the database of the storage device 23. Screen G7 in FIG. 11 is provided with a text box R73 for inputting the characteristic name, a text box R74 for inputting the lower limit of the characteristic value, a text box R75 for inputting the upper limit of the characteristic value, and a text box R76 for inputting the units. A search button B72 is also provided. This allows the user to search for raw material names by specifying the product name, the chemical formula of the raw material, or the structure of the raw material and by pressing the search button B72. The user can also search for raw material names by specifying the characteristic name and the range of the characteristic value and by pressing the search button B72.

When the search button B72 is pressed, the screen transitions to screen G8. Screen G8 displays the names of raw materials found as a result of the search. The raw material with this raw material name is the target raw material for which the user is searching for potential sales destinations. In order to search for potential buyers of this raw material, screen G8 is provided with a potential sales destination button B73. When the potential sales destination button B73 is pressed, the screen transitions to screen G9 in FIG. 12. Screen G9 displays a list of potential sales destinations for the raw material (hereinafter also referred to as a list of promising sales destinations).

The list of promising sales destinations may include, for example, candidate companies for the sales target, a patent publication number (or patent number) which is an example of information identifying a patent document in which an working example of the target raw material to be replaced is described, the raw material to be replaced by the target raw material, characteristic values of the product to be replaced (also referred to as characteristic values before replacement), and characteristic values after replacement with the target raw material (also referred to as characteristic values after replacement).

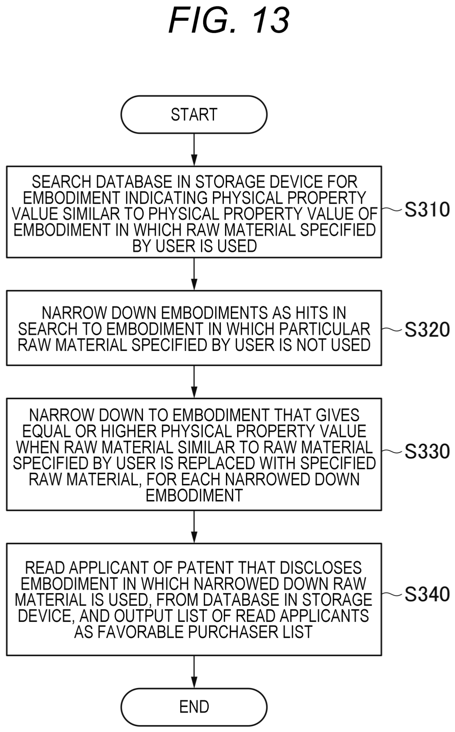

The process flow for outputting a list of candidate sales destinations when the candidate sales destination button is pressed will be described below with reference to Fig. 13. Fig. 13 is a flowchart showing an example of the process flow when the candidate sales destination button according to the third embodiment is pressed. Here, as an example of the process, an example of extracting only examples that do not use raw materials specified by the user will be described.

(Step S310) The processor 26 searches the database in the storage device 23 for working examples that exhibit similar physical property values to the physical property values of the working examples in which the raw material designated by the user is used.

(Step S320) As an example, the processor 26 narrows down the working examples found in the search to working examples that do not use a specific raw material designated by the user. Alternatively, the processor 26 may extract examples that do not use a specific raw material designated by the user during the search.

(Step S330) For each of the narrowed down examples, the processor 26 narrows down the raw materials that are similar to the raw material specified by the user (for example, in the product database, those that belong to the same category or those that have a certain degree of similarity in chemical fingerprints) to those that have the same or greater physical property values (or similar physical property values) when replaced with the specified raw material.

Here, when narrowing down the raw materials whose physical property values are equal to or greater than those of the raw material specified by the user, the machine learning model constructed in the first embodiment may be used. Specifically, for example, when narrowing down the raw materials whose physical property values are similar when the target raw material whose raw material name is input by the user is replaced with other raw material, the processor 26 may input a pair of the other raw material and the amount of the other raw material into the trained machine learning model of the first embodiment to determine predicted values of the physical property values of the prediction target physical property name, and compare each of the predicted values of the physical property values with the physical property values of the target raw material to extract raw materials whose predicted physical property values are equal to or greater than (or similar to) physical property values of the raw material specified by the user.

(Step S340) The processor 26 reads out the applicants (or patent holders) of the patent documents in which working examples using the narrowed-down raw materials are described from the database of the storage device 23, and outputs the list of the read out applicants (or patent holders) as a list of potential sales destinations (a list of promising sales destinations) of the target raw materials. This list of promising sales destinations may include the raw materials described in the extracted working examples as products to be replaced.

As described above, the information processing system according to the third embodiment comprises at least one storage device in which working examples of patent documents are stored, and at least one processor. The processor extracts working examples stored in the storage device that use raw materials specified by the user and/or working examples that do not use the input raw materials among raw materials with similar physical property values realized by the combination of the raw materials, and outputs information for displaying a list of applicants or patent holders of patent documents in which the extracted working examples are described. This list may include similar raw materials described in the extracted working examples as target raw materials to be replaced.

According to this configuration, the applicants listed in the patent documents in which the raw material specified by the user or a raw material similar to the raw material is described in the working examples are output, making it easier to search for companies that may be able to use the target raw material.

Furthermore, after extracting the working examples, the processor may narrow down the list to raw materials that have similar physical property values when the target raw material designated by the user is replaced with other raw material.

In addition, when narrowing down the target raw material specified by the user to raw materials whose physical property values are similar when replaced with other raw materials, the processor may input pairs of the other raw materials and the amounts of the other raw materials into the trained machine learning model of the first embodiment, thereby determining predicted values of the physical property values of the predicted target physical property name, and compare each of the predicted physical property values with the physical property values of the target raw material, thereby extracting raw materials whose predicted physical property values are equal to or greater than or similar to the physical property values of the raw material specified by the user.

### <Modification example>

In the above-described embodiment, the input to the first machine learning model is a SMILES character string or chemical graph structure data corresponding to each of the raw materials and the amount of each of the raw materials, but this is not limited, and may be a product name or substance name and the amount of each of the raw materials. In other words, at least one of a SMILES character string, chemical graph structure data, product name, or substance name corresponding to each of the raw materials and the amount of each of the raw materials may be input to the first machine learning model. In this case, the first machine learning model is a model in which parameters are adjusted so that the output can be predicted from the input using a learning data set in which at least one of a SMILES character string, chemical graph structure data, product name, or substance name corresponding to each of the raw materials and the amount of each of the raw materials are input, and the physical property value of the prediction target physical property name is output.

Similarly, the input to the second machine learning model is a set of numerical values based on the chemical fingerprint, SMILES character string, or chemical graph structure data corresponding to each of the raw materials and the amount of each of the raw materials, but is not limited to this, and may be a set of numerical values based on the product name or substance name and the amount of each of the raw materials. That is, a set of numerical values based on at least one of the chemical finger print, SMILES character string, chemical graph structure data, product name, or substance name corresponding to each of the raw materials and the amount of each of the raw materials may be input to the second machine learning model.

In this case, the second machine learning model is a trained model in which a set of numerical values obtained by converting at least one of the chemical fingerprints, SMILES strings, or chemical graph structure data, or product names or substance names corresponding to each raw material and the amounts of each raw material are used as input, and parameters are adjusted so that the output can be predicted from the input using a learning data set in which the physical property value of the target physical property name to be predicted is output.

In the above-described embodiment, the first machine learning model or the second machine learning model is constructed for each prediction target property name, but the present invention is not limited to this. The first machine learning model or the second machine learning model may be constructed by collectively learning a plurality of properties (e.g., cleaning power of a detergent, surface roughness caused by an abrasive liquid, etc.) of raw materials used in a plurality of fields.

In the above-described embodiment, as an example, the raw materials and additives are separated and described as the first chemical fingerprint and the second chemical fingerprint, respectively. However, this is not limited to the above, and the raw materials may contain additives. In this case, each raw material has a raw material type (kind) such as a polymer, a polymerization initiator, a flame retardant, etc., and the input to the first machine learning model or the second machine learning model may include the type of raw material or a set of values based on the type (e.g., a numeric string, specifically, for example, a vector). In this case, at least one processor may further input the type of raw material or the set of values based on the type acquired from the user to the first machine learning model or the second machine learning model, thereby acquiring a predicted value of the physical property value of the predicted target physical property name for a composition composed of each of the raw materials.

The input to the first machine learning model or the second machine learning model may further include a physical property of the raw material (e.g., fiber length) or a group of numerical values based on the physical property (e.g., a numeric string, specifically, for example, a vector). In that case, the processor may obtain a predicted value of the characteristic value of the physical property name to be predicted by further inputting the physical property of the raw material obtained from the user (e.g., fiber length) or a group of numerical values based on the physical property into the first machine learning model or the second machine learning model.

The input to the first or second machine learning model may include the features of the raw material (e.g., higher-order structure in the case of a polymer) or a set of numerical values based on the features (e.g., a numerical sequence, specifically, for example, a vector). In that case, the processor may obtain a predicted value of the physical property value of the predicted target physical property name by further inputting the features of the raw material (e.g., higher-order structure in the case of a polymer) or a set of numerical values based on the features acquired from the user into the first or second machine learning model. The features may be described in text, in which case the text may be converted into a numerical sequence (e.g., a vector) and input.

The input of the first machine learning model or the second machine learning model may include information on manufacturing process conditions and/or equipment used in the process (e.g., facilities, machines, or devices, etc.) or a group of numerical values based on the information (e.g., a numerical sequence, specifically, for example, a vector). The process conditions may include, for example, at least one of the conditions during manufacturing (e.g., conditions when mixing raw materials, specifically, temperature, pressure, and their time series changes, maintenance time, or time change rate, etc.), the name of a measuring device, the measurement conditions, and the measurement standards. In addition, the information on the equipment used in the process may include the name of the manufacturing equipment (e.g., a machine for mixing raw materials, etc.) or the specifications of the manufacturing equipment (e.g., the nozzle diameter of a 3D printer, etc.). In that case, the processor may obtain a predicted value of the physical property value of the predicted target physical property name for a composition composed of each of the raw materials by further inputting the process conditions and/or information on the equipment used in the process acquired from the user into the first machine learning model or the second machine learning model.

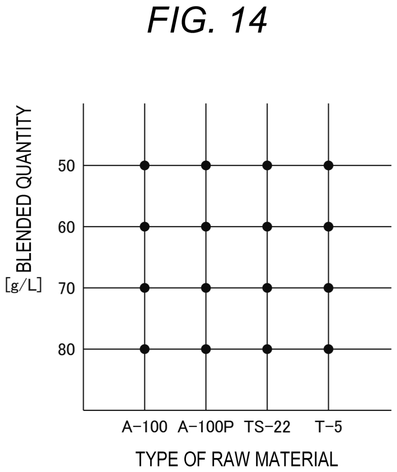

FIG. 14 is a diagram explaining a grid search of raw material types and blend amounts. In FIG. 14, for example, the blend amounts for each raw material type are divided into grids in 10% increments, and each grid corresponds to a pair of raw material type and blend amount. For simplicity, in FIG. 14, the search range for a grid search when one type of raw material is selected is shown in two dimensions, but if multiple raw materials are selected, the grid search range will increase accordingly. For example, if there are two types of raw materials, the grid search range will be four-dimensional.

For example, at least one processor may obtain target physical property values specified by the user and obtain the range of blend amounts specified by the user.

In this case, at least one processor may determine multiple candidate blend amounts within the blend amount range specified by the user for each candidate raw material, and predict physical property values for all combinations of the candidate raw material set and each candidate blend amount of the candidate raw material. Here, the candidate raw materials may be obtained by the processor as each candidate raw material specified by the user. Alternatively, the processor may obtain a substance category (e.g., thermoplastic resin, etc.) specified by the user, and each raw material belonging to the substance category specified by the user may be extracted as the candidate raw material. Here, the candidate blend amounts may be selected in predetermined increments as shown in FIG. 14, and in this case, all combinations of the candidate raw material set and each candidate blend amount of the candidate raw material may be determined by a so-called grid search.

In this case, at least one processor may output the candidate raw material set and each amount of the candidate raw material based on a comparison between the target physical property value and each predicted physical property value. Specifically, for example, the at least one processor may output the set of candidate raw materials and the respective amounts of the candidate raw materials in order of proximity of the predicted physical property value to the target physical property value.

### <Fourth Embodiment>

Next, a fourth embodiment will be described. In the fourth embodiment, when a user selects a working example in a patent document and specifies one or more target raw materials among the raw materials constituting the composition of the selected working example, one or more candidate raw materials and the range of the blending amounts of the candidate raw materials, at least one processor outputs predicted values of physical properties for each candidate blending amount included in the specified range when the target raw material is changed to each candidate raw material. The objective is to make it easy to obtain an optimal combination of raw materials that satisfy the material properties that a user desired to obtain and the blending amounts of the raw materials.

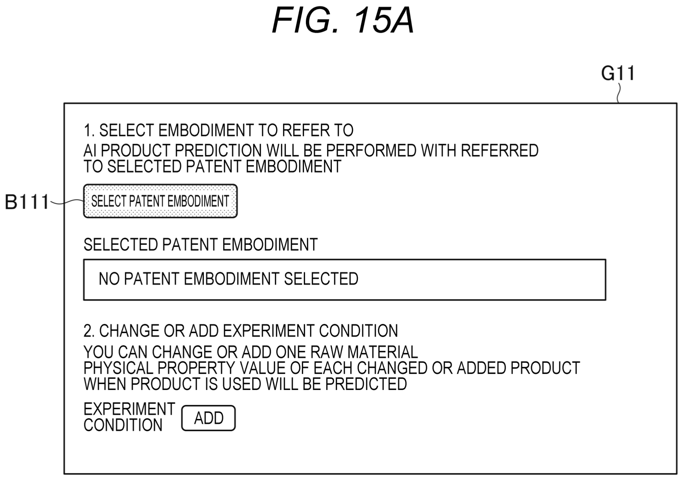

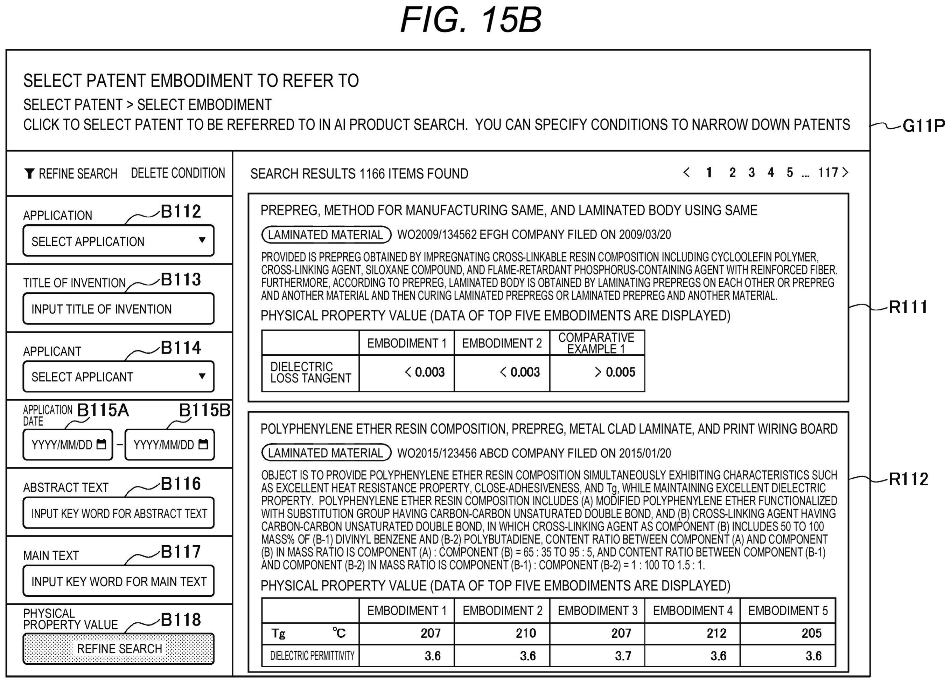

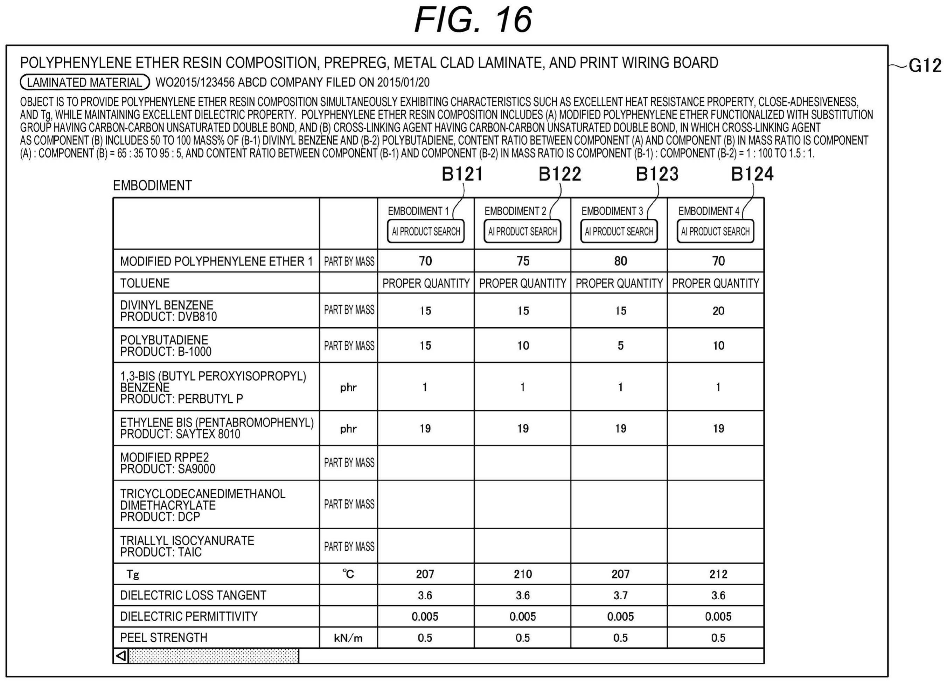

The screen transitions of the terminal will be explained using examples of terminal screens in Figures 15 to 19. Figure 15A is a diagram showing a working example of a terminal screen in the fourth embodiment. As shown in Figure 15A, screen G11 shows a "Select a patent working example" button B111 for selecting a working example to reference. When this "Select a patent working example" button B111 is pressed, a patent document search screen (not shown) is displayed, for example as a pop-up. Figure 15B is a diagram showing an example of the patent document search screen in the fourth embodiment. For example, information about patent documents may be collected for each use application, in which case information about patent documents may be stored in storage device 23 for each application of the technology related to the patent document (e.g., lithium ion batteries, sealing materials, or cosmetics), or information about patent documents and the use application of the technology related to the patent document may be associated with each other and stored in storage device 23. Here, information about patent documents includes, for example, information that identifies the patent document (e.g., application number and/or publication number and/or patent number), the title of the invention, the applicant, the filing date, an abstract, the main text, the names of physical properties for each working example, and a set of their physical property values. In this case, the patent document can be searched by specifying conditions such as keyword search on the patent document search screen. Specifically, for example, on the patent document search screen G11P, a select box B112 for selecting the use, a text box B113 for inputting a keyword to search within the title of the invention, a select box B114 for selecting the applicant, a text box B115A for inputting the start date of the range of application dates, a text box B115B for inputting the end date of the range of application dates, a text box B116 for inputting a keyword to search within the abstract text, and a text box B117 for inputting a keyword to search within the main text are displayed. In addition, the patent document search screen G11P may be capable of specifying the name of a physical property, or a combination of the name of a physical property and the range of its physical property value. When the "Refine" button B118 is pressed on the patent document search screen G11P, a search is performed with the set conditions, and the search results are displayed. Here, patent documents are shown in the screen areas R111 and R112 as examples of search results. On this patent document search screen, when, for example, one patent document (here, as an example, the patent document in the screen region R112) is selected, the screen transitions to a screen G12 in FIG.16.

FIG. 16 is an example of a working example selection screen in the fourth embodiment. In screen G12 in FIG. 16, information about the selected patent document (e.g., title of the invention, publication number, name of the applicant, filing date, abstract, etc.) is displayed. In screen G12 in FIG. 16, for each working example described in the selected patent document, the names of the raw materials constituting the composition (e.g., a polyphenylene ether resin composition) and the amounts of each of the raw materials mixed are displayed, and the properties at that time (e.g., glass transition temperature Tg, dielectric constant, dielectric tangent, tensile strength, and in the case of a cosmetic example, moisturizing properties and hair gloss, etc.) are displayed.

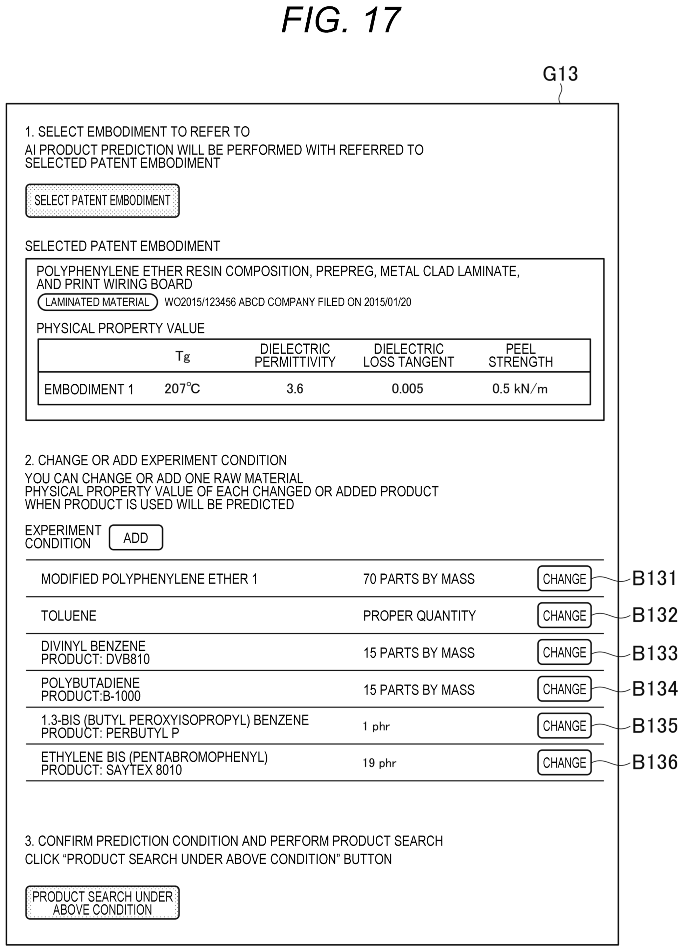

In the screen G12 of Fig. 16, "AI product search" buttons B121, B122, B123, and B124 are shown as examples associated with the respective working examples. When one of these buttons is pressed, the screen transitions to the screen G13 of Fig. 17. Here, as an example, the case where the "AI product search" button B121 associated with working example 1 of the selected patent document is pressed will be described below.

FIG. 17 is an example of a screen that appears when the "AI product search" button B121 associated with working example 1 of the selected patent document is pressed on screen G12 of FIG. 16. On screen G13 of FIG. 17, "Change" buttons B131-B136 are shown associated with each of the raw materials that make up a composition (e.g., a polyphenylene ether resin composition). By pressing these "Change" buttons B131-B136, the user can change the corresponding raw material to other raw material and/or change the blending amount. Below, as an example, a case where, for example, the "Change" button B131 is pressed on screen G13 of FIG. 17 will be described.

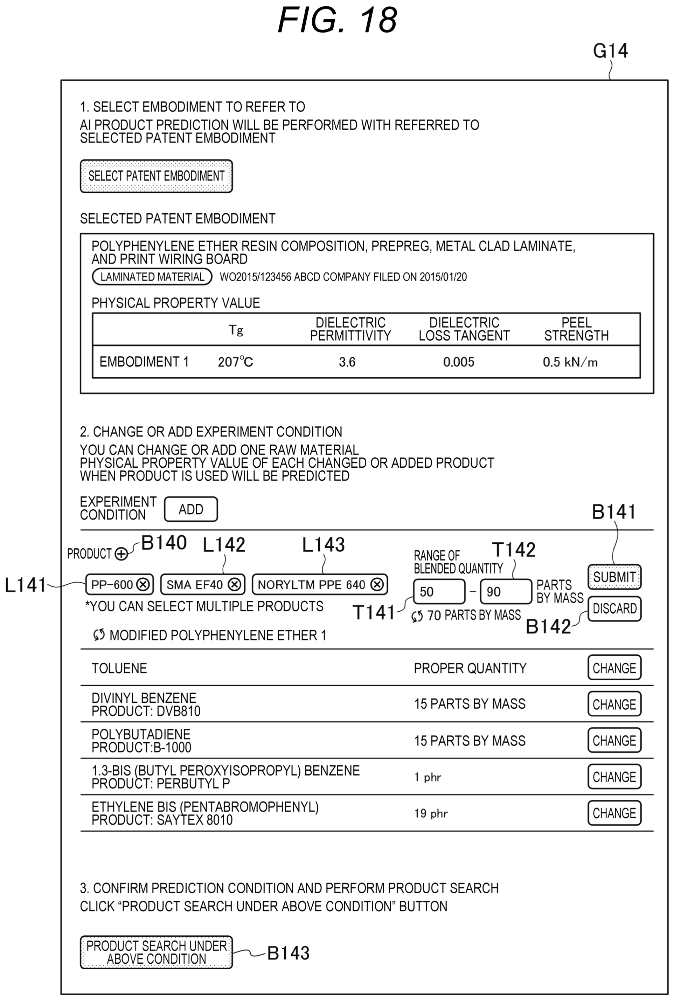

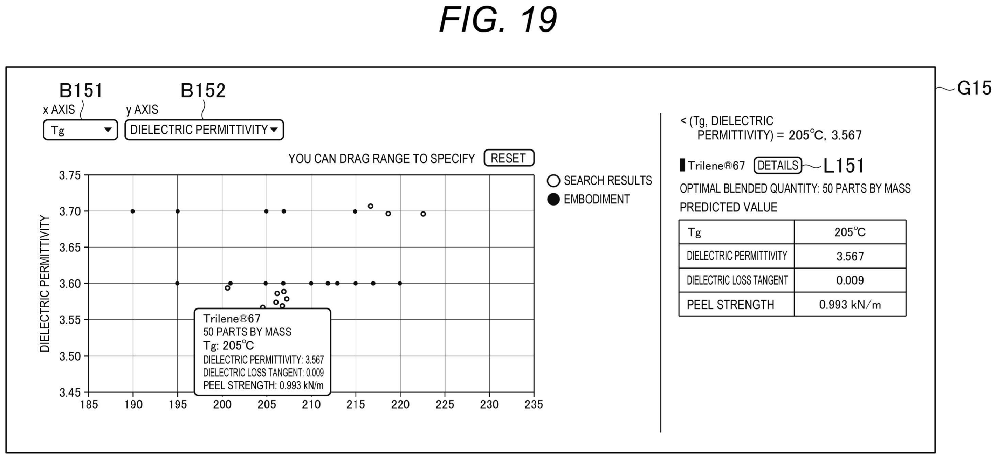

FIG. 18 is an example of a screen when, for example, the "Change" button B131 is pressed on the screen G13 of FIG. 17. In the screen G14 of FIG. 18, a plus button associated with "Product" is shown, and when the plus button is pressed, a candidate raw material selection screen is displayed, for example, as a pop-up. When the user selects a candidate raw material on this candidate raw material selection screen, the candidate raw material (here, as an example, the product name) is displayed as a label on the screen G14. In the example of the screen G14, "PP-600", "SMA-EF-40", and "NORYLTM PPE 640" are selected by the user as candidate raw materials, and as a result, a label L141 for "PP-600", a label L142 for "SMA-EF-40", and a label L143 for "NORYLTM PPE 640" are displayed. Also shown are a text box T141 for inputting the lower limit of the blending amount of these candidate raw materials, and a text box T142 for inputting the upper limit of the blending amount of these candidate raw materials. Also displayed are a "Confirm" button B141 and a "Cancel" button B142, and when the "Confirm" button B141 is pressed, the selected candidate raw materials and blending amount range are confirmed. On the other hand, when the "Cancel" button B142 is pressed, the selected candidate raw materials and blending amount range are cancelled. When the "Search for product with the above conditions" button B143 is pressed on screen G14, the screen transitions to a screen showing the predicted results of the physical property values, for example, as shown in Figure 19.

Although an example in which the user directly specifies candidate raw materials has been described, this is not limiting, and the user may specify a substance category (e.g., thermoplastic resin). In this case, at least one processor (e.g., processor 26) may determine candidate raw materials belonging to the substance category (e.g., thermoplastic resin) specified by the user. Specifically, for example, substance categories may be stored in association with identification information that identifies raw materials in storage device 23, and at least one processor (e.g., processor 26) may determine candidate raw materials by referring to storage device 23 and reading out raw materials corresponding to the substance category specified by the user from storage device 23 as candidate raw materials.

FIG. 19 is an example of a screen showing the predicted results of physical properties in the fourth embodiment. Screen G15 in FIG. 19 shows a graph showing predicted physical properties for all combinations of candidate raw materials and their respective candidate amounts determined by grid search in the candidate raw materials and their respective candidate amounts range set in FIG. 18. Screen G15 in FIG. 19 shows a select box B151 for selecting the horizontal axis (x-axis) of the graph and a select box B152 for selecting the vertical axis (y-axis) of the graph. In this example, the vertical axis (y-axis) of the graph is the dielectric constant, and the horizontal axis (x-axis) is the glass transition temperature Tg. On the graph in screen G15 in FIG. 19, as an example, the physical properties of a working example are plotted, and predicted values of physical properties are plotted for each combination of the candidate raw materials and the respective candidate amounts of the candidate raw materials. This allows the user to compare predicted values of physical properties, allowing the user to select the optimal combination of candidate raw materials and their amounts. For example, when the mouse is placed over one of the plots in the graph, information including the name (e.g., product name or substance name) of the candidate raw material corresponding to the plot over which the mouse is placed, the blending amount, and predicted values of the physical properties (e.g., glass transition temperature Tg, dielectric constant, dielectric tangent, peel strength) is displayed as a pop-up as an example, and also, as an example, on the right side of screen G15, the name of the candidate raw material corresponding to the plot over which the mouse is placed, the blending amount, predicted values of the physical properties, and a link L151 labeled "Details" are displayed. When this link L151 is pressed, details of the candidate raw material are displayed.

To output screen G15 of FIG. 19, the following steps may be executed. At least one processor (e.g., processor 26) determines a plurality of candidate values for the amount of another raw material in a range specified by the user when a part of the raw materials constituting the target composition is replaced with another raw material specified by the user. Here, the target composition is, for example, one described in a working example selected by the user in a patent document selected by the user.

At least one processor (e.g., processor 26) determines predicted values of physical properties for all of the determined plurality of candidate values for amount. Then, at least one processor (e.g., processor 26) outputs information for presenting predicted values of physical properties in association with each of the plurality of candidate values for amount.

Here, in the step of determining the predicted value of the physical property, at least one processor (e.g., processor 26) obtains the predicted value of the physical property by inputting at least one of a SMILES character string, chemical graph structure data, product name, or substance name corresponding to each of the raw materials and the amount of each of the raw materials to a first machine learning model, or by inputting a group of numerical values based on at least one of a chemical fingerprint, a SMILES character string, chemical graph structure data, product name, or substance name corresponding to each of the raw materials and the amount of each of the raw materials to a second machine learning model.

Here, the first machine learning model is a model in which parameters are adjusted so that the output can be predicted from the input using a learning data set in which at least one of the SMILES character string, chemical graph structure data, product name, or substance name corresponding to each raw material and the amount of each raw material are input, and the physical property value of the prediction target physical property name is output.

The second machine learning model is a model in which parameters are adjusted so that the output can be predicted from the input using a learning data set in which at least one of the SMILES character string, chemical graph structure data, product name, or substance name corresponding to each raw material and the amount of each raw material is input, and the physical property value of the prediction target physical property name is output. According to this configuration, the user can compare the predicted physical property values, so that the optimal combination of candidate raw materials and blending amounts can be selected.

At least a part of the computer system 2 described in the above embodiment may be configured with hardware or software. When configured with software, a program that realizes at least a part of the functions of the computer system 2 may be stored in a computer-readable recording medium and read and executed by a computer. The recording medium is not limited to removable ones such as magnetic disks and optical disks, but may be fixed recording media such as hard disk drives and memories.

In addition, a program for realizing at least a part of the functions of the computer system 2 may be distributed via a communication line (including wireless communication) such as the Internet. Furthermore, the program may be encrypted, modulated, or compressed and distributed via a wired line or wireless line such as the Internet, or stored on a recording medium.

Furthermore, the computer system 2 may be operated by one or more information devices. When multiple information devices are used, one of the devices may be a computer, and the computer may execute a predetermined program to realize the functions of at least one of the means of the computer system 2.

In the method invention, all the steps may be realized by automatic control using a computer. Alternatively, each step may be performed by a computer while the progress between steps is controlled manually. Furthermore, at least some of the steps may be performed manually.

As described above, the present invention is not limited to the above-described embodiment as it is, and in the implementation stage, the components can be modified and embodied without departing from the gist of the invention. In addition, various inventions can be formed by appropriately combining the multiple components disclosed in the above-described embodiment. For example, some components may be deleted from all the components shown in the embodiment. Furthermore, components from different embodiments may be appropriately combined.

### Reference Signs List

S Information processing system
1 Terminal
11 Input interface
12 Communication module
13 Storage device
14 Memory
15 Output interface
16 Processor
17 Display
2 Information processing system
21 Input interface
22 Communication module
23 Storage device
24 Memory
25 Output interface
26 Processor

## Claims

1. An information processing system comprising:
at least one processor obtaining a combination of information identifying each of the raw materials received from the user and the amount of each of the raw materials, and obtaining a predicted value of a physical property of the property name to be predicted for a composition comprising each of the raw materials by inputting into a first machine learning model at least one of the chemical fingerprints, SMILES strings or chemical graph structure data or product name or substance name corresponding to each of the raw materials and the amount of each of the raw materials, or by inputting into a second machine learning model a set of values based on at least one of the chemical fingerprints, SMILES strings or chemical graph structure data or product name or substance name corresponding to each of the raw materials and the amount of each of the raw materials,
wherein the first machine learning model is a model in which parameters are adjusted so that it can predict outputs from inputs by means of a learning data set in which at least one of the chemical fingerprints, SMILES strings or chemical graph structure data or product names or substance names corresponding to each of the raw materials and the amount of each of the said raw materials are input, and physical property value of the target property name to be predicted is output, and the second machine learning model is a model in which parameters are adjusted so that it can predict outputs from inputs by means of a learning data set in which a group of numerical values based on at least one of the chemical fingerprints, SMILES strings or chemical graph structure data or product names or substance names corresponding to each of the raw materials and the amount of each of the raw materials is input, and physical property value of the target property name to be predicted is output.

2. The information processing system according to claim 1, wherein the first machine learning model or the second machine learning model is constructed by collectively learning a plurality of physical properties of raw materials used in a plurality of fields.

3. The information processing system according to claim 1 or 2, wherein the raw materials include additives, and the input of the first machine learning model or the second machine learning model includes the type of raw material or a group of numerical values based on the type, and the processor further inputs the type of raw material or the group of numerical values based on the type acquired from the user into the first machine learning model or the second machine learning model, thereby obtaining a predicted value of the physical property value of the prediction target physical property name for a composition composed of each of the raw materials.

4. The information processing system according to any one of claims 1 to 3, wherein the input of the first machine learning model or the second machine learning model further includes the physical properties of the raw materials or a group of numerical values based on the physical properties, and the processor further inputs the physical properties of the raw materials or a group of numerical values based on the physical properties obtained from the user into the first machine learning model or the second machine learning model, thereby obtaining a predicted value of the characteristic value of the prediction target physical property name for a composition composed of each of the raw materials.

5. The information processing system according to any one of claims 1 to 4, wherein the input of the first machine learning model or the second machine learning model includes the characteristics of raw materials or a group of numerical values based on the characteristics, and the processor further inputs the characteristics of the raw materials or the group of numerical values based on the characteristics obtained from the user into the first machine learning model or the second machine learning model, thereby obtaining a predicted value of the physical property value of the prediction target physical property name for a composition composed of each of the raw materials.

6. The information processing system according to any one of claims 1 to 5, wherein the input of the first machine learning model or the second machine learning model includes information about process conditions and/or equipment used in the process or a group of numerical values based on the information, and the processor further inputs the information about the process conditions and/or equipment used in the process acquired from the user or a group of numerical values based on the information into the first machine learning model or the second machine learning model, thereby obtaining a predicted value of the physical property value of the prediction target physical property name for a composition composed of each of the raw materials.

7. The information processing system according to any one of claims 1 to 6, wherein the processor extracts at least one raw material candidate based on at least one of the raw material product name, raw material manufacturer name, raw material category, and raw material uses received from the user, and outputs information for displaying the extracted raw material candidate in a selectable manner.

8. The information processing system comprising:
at least one processor,
wherein the processor creates a child population of the next generation according to a genetic algorithm by regarding a combination of raw materials and the amounts of each of the raw materials as a parent population,
calculates a fitness function value using the combination of raw materials and the amounts of each of the raw materials indicated by the created child population of the next generation,
repeates the process of creating a child population of the next generation until a termination condition based on a physical property value range specified by the user is satisfied, and when the termination condition is satisfied, outputs information including the combination of raw materials indicated by each of the child populations of the next generation created last and the amounts of each of the raw materials.

9. The information processing system according to claim 8, wherein the processor acquires at least one set of desired property value ranges input by a user for a desired property name, and inputs at least one of a chemical fingerprint, a SMILES character string, or chemical graph structure data, or a product name or a substance name corresponding to each of the raw materials included in the initial combination of raw materials and an amount of each of the raw materials into a first machine learning model, or inputs a group of numerical values based on at least one of a chemical fingerprint, a SMILES character string, or chemical graph structure data, or a product name or a substance name corresponding to each of the raw materials and an amount of each of the raw materials into a second machine learning model, thereby acquiring a predicted value of a property value of the physical property name,
narrows down the obtained predicted values of the physical properties to combinations of raw materials and the amounts of each of the raw materials that satisfy the desired range of the physical properties specified by the user,
considers the narrowed down combinations of raw materials and the amounts of each of the raw materials as a parent population, and creates a child population of the next generation according to a genetic algorithm,
the first machine learning model is a model in which parameters are adjusted so that the output can be predicted from the input using a learning data set in which at least one of the chemical fingerprints, SMILES character strings, chemical graph structure data, product names, or substance names corresponding to each of the raw materials and the amounts of each of the raw materials are input, and the physical property value of the prediction target physical property name is output,
the second machine learning model is a model in which parameters are adjusted so that an output can be predicted from an input using a learning data set in which a group of numerical values obtained by converting at least one of a chemical fingerprint, a SMILES character string, a chemical graph structure data, a product name, or a substance name corresponding to each raw material and the amount of each raw material is input, and a physical property value of a physical property name is output.

10. The information processing system according to claim 9, wherein the first machine learning model or the second machine learning model is constructed for each physical property name, and the processor obtains a predicted value of the physical property value of the prediction target physical property name by using the first machine learning model or the second machine learning model corresponding to the physical property name received from the user.

11. The information processing system according to claim 9 or 10, wherein the processor further outputs a predicted value of the property value of the desired property name exhibited by each of the offspring population of the next generation finally produced.

12. The information processing system according to any one of claims 8 to 11, wherein when one of the outputted raw materials is selected by the user, the processor outputs contact information of a sales company that sells the raw material and/or detailed information of the raw material.

13. The information processing system comprising:
at least one storage device in which data relating to working examples of patent documents are stored; and
at least one processor,
wherein the processor extracts, from among the working examples stored in the storage device, a working example that use raw materials designated by a user and/or a working example that do not use the input raw materials among raw materials with similar physical property values realized by the combination of the raw materials, and outputs information for displaying a list of applicants or patent holders of patent documents in which the extracted working examples are described.

14. The information processing system according to claim 13, wherein after extracting the working examples, the processor narrows down the working examples to raw materials that have similar physical property values when the target raw material designated by the user is replaced with other raw material.

15. The information processing system according to claim 14, wherein when narrowing down raw materials whose physical property values are similar when a target raw material designated by a user is replaced with other raw material, the processor inputs a pair of the other raw material and the amount of the other raw material into a trained machine learning model of a first embodiment to determine predicted values of the physical property values of the predicted target physical property name, and compares each of the predicted values of the physical property values with the physical property values of the target raw material to extract raw materials whose predicted physical property values are equal to or greater than or similar to the physical property values of the raw material designated by the user.

16. The information processing system according to any one of claims 13 to 15, wherein the list includes similar ingredients described in the extracted examples as ingredients to be substituted.

17. The information processing system comprising:
at least one processor,
wherein the at least one processor acquires target physical property values specified by a user,
acquires a range of blend amounts specified by the user,
determines multiple candidate blend amounts within the range of blend amounts specified by the user for each of the candidate raw materials, predicts physical property values for all combinations of a set of candidate raw materials and each of the candidate blend amounts of the candidate raw materials, and
outputs the set of candidate raw materials and each of the candidate raw materials based on a comparison between the target physical property values and each of the predicted physical property values.

18. The information processing system according to claim 17, wherein the at least one processor obtains a substance category designated by a user, and extracts each of the raw materials belonging to the substance category designated by the user as the candidate raw materials.

19. The information processing system comprising:
at least one processor,
wherein the at least one processor executes the steps of:
determining a plurality of candidate values for the amount of other raw material in a range specified by a user when a portion of the raw materials constituting a target composition is replaced with other raw material specified by a user;
determining predicted values of physical properties for all of the determined plurality of candidate values for the amount; and
outputting information for presenting predicted values of physical properties in correspondence with each of the plurality of candidate values for the amount,
in the step of determining the predicted value of the physical property, the at least one processor inputs at least one of a SMILES character string, a chemical graph structure data, a product name, or a substance name corresponding to each of the raw materials and an amount of each of the raw materials to a first machine learning model, or inputs a group of numerical values based on at least one of a chemical fingerprint, a SMILES character string, a chemical graph structure data, a product name, or a substance name corresponding to each of the raw materials and an amount of each of the raw materials to a second machine learning model, thereby obtaining a predicted value of the physical property,
the first machine learning model is a model in which parameters are adjusted so that the output can be predicted from the input using a learning data set in which at least one of a SMILES character string, chemical graph structure data, product name, or substance name corresponding to each raw material and the amount of each raw material are input, and the physical property value of the prediction target physical property name is output,
the second machine learning model is a model in which parameters are adjusted so that an output can be predicted from an input using a learning data set in which a group of numerical values obtained by converting at least one of a chemical fingerprint, a SMILES character string, or chemical graph structure data, or a product name or a substance name corresponding to each raw material and the amount of each raw material is input, and a physical property value of a prediction target physical property name is output.

20. A program for causing a computer capable of referring to at least one storage device that stores
a first machine learning model is a model in which parameters are adjusted so that it can predict outputs from inputs by means of a learning data set in which at least one of the chemical fingerprints, SMILES strings or chemical graph structure data or product names or substance names corresponding to each of the raw materials and the amount of each of the said raw materials are input, and physical property value of the target property name to be predicted is output, or
a second machine learning model is a model in which parameters are adjusted so that it can predict outputs from inputs by means of a learning data set in which a group of numerical values based on at least one of the chemical fingerprints, SMILES strings or chemical graph structure data or product names or substance names corresponding to each of the raw materials and the amount of each of the raw materials is input, and physical property value of the target property name to be predicted is output,
to execute obtaining a combination of information identifying each of the raw materials received from the user and the amount of each of the raw materials, and obtaining a predicted value of a physical property of the property name to be predicted for a composition comprising each of the raw materials by inputting into a first machine learning model at least one of the chemical fingerprints, SMILES strings or chemical graph structure data or product name or substance name corresponding to each of the raw materials and the amount of each of the raw materials, or by inputting into a second machine learning model a set of values based on at least one of the chemical fingerprints, SMILES strings or chemical graph structure data or product name or substance name corresponding to each of the raw materials and the amount of each of the raw materials.

21. A program for causing a computer to execute
creating a child population of the next generation according to a genetic algorithm by regarding a combination of raw materials and the amounts of each of the raw materials as a parent population
calculating a fitness function value using the combination of raw materials and the amounts of each of the raw materials indicated by the created child population of the next generation,
repeating the process of creating a child population of the next generation until a termination condition based on a physical property value range specified by the user is satisfied, and when the termination condition is satisfied, outputting information including the combination of raw materials indicated by each of the child populations of the next generation created last and the amounts of each of the raw materials.

22. A program for causing a computer capable of referring to at least one storage device in which data related to a working example of the patent document is stored, to execute
extracting, from among the working examples stored in the storage device, a working example that use raw materials designated by a user and/or a working example that do not use the input raw materials among raw materials with similar physical property values realized by the combination of the raw materials, and
outputting information for displaying a list of applicants or patent holders of patent documents in which the extracted working examples are described.

23. A program for causing a computer to execute
acquiring target physical property values specified by a user,
acquiring a range of blend amounts specified by the user,
determining multiple candidate blend amounts within the range of blend amounts specified by the user for each of the candidate raw materials, predicts physical property values for all combinations of a set of candidate raw materials and each of the candidate blend amounts of the candidate raw materials, and
outputting the set of candidate raw materials and each of the candidate raw materials based on a comparison between the target physical property values and each of the predicted physical property values.

24. A program for causing a computer to execute
determining a plurality of candidate values for the amount of other raw material in a range specified by a user when a portion of the raw materials constituting a target composition is replaced with other raw material specified by a user;
determining predicted values of physical properties for all of the determined plurality of candidate values for the amount; and
outputting information for presenting predicted values of physical properties in correspondence with each of the plurality of candidate values for the amount,
in the step of determining the predicted value of the physical property, the at least one processor inputs at least one of a SMILES character string, a chemical graph structure data, a product name, or a substance name corresponding to each of the raw materials and an amount of each of the raw materials to a first machine learning model, or inputs a group of numerical values based on at least one of a chemical fingerprint, a SMILES character string, a chemical graph structure data, a product name, or a substance name corresponding to each of the raw materials and an amount of each of the raw materials to a second machine learning model, thereby obtaining a predicted value of the physical property,
the first machine learning model is a model in which parameters are adjusted so that the output can be predicted from the input using a learning data set in which at least one of a SMILES character string, chemical graph structure data, product name, or substance name corresponding to each raw material and the amount of each raw material are input, and the physical property value of the prediction target physical property name is output,
the second machine learning model is a model in which parameters are adjusted so that an output can be predicted from an input using a learning data set in which a group of numerical values obtained by converting at least one of a chemical fingerprint, a SMILES character string, or chemical graph structure data, or a product name or a substance name corresponding to each raw material and the amount of each raw material is input, and a physical property value of a prediction target physical property name is output.
